# NEW EUROPEAN PATENT SPECIFICATION

(11) **EP 3 383 385 B2**
(45) Date of publication and mention of the opposition decision: **06.09.2023**
(45) Mention of the grant of the patent: 29.07.2020
(21) Application number: 16805792.5
(22) Date of filing: 01.12.2016
(51) Int. Cl.: A61K 31/223, A61K 31/573

(54) **MELFLUFEN DOSAGE REGIMENS FOR CANCER**
MELFLUFENDOSIERUNGSPLÄNE FÜR KREBS
RÉGIMES POSOLOGIQUES DE MELFLUFEN POUR LE CANCER

(30) Priority: 01.12.2015 GB 201521217
(43) Date of publication of application: 10.10.2018
(60) Divisional application: 20187064.9
(73) Proprietor: Oncopeptides AB, 111 53 Stockholm (SE)
(72) Inventor: LINDBERG, Jakob, S-111 53 Stockholm (SE)
(74) Representative: Abel & Imray LLP
(86) International application number: PCT/EP2016/079511
(87) International publication number: WO 2017/093443

(56) References cited:
- WO-A1-2014/065751
- US-A1- 2014 302 014
- MAGAROTTO V ET AL: "ENCOURAGING PRELIMINARY DATA IN ONGOING OPEN-LABEL PHASE 1/2 STUDY OF SAFETY AND EFFICACY OF MELFLUFEN AND *DEXAMETHASONE FOR PATIENTS WITH RELAPSED AND RELAPSED-REFRACTORY MULTIPLE MYELOMA", HAEMATOLOGICA, THE HEMATOLOGY JOURNAL : OFFICIAL ORGAN OF THE EUROPEAN HEMATOLOGY ASSOCIATION, FONDAZIONE FERRATA STORTI, IT, vol. 100, no. Suppl.1, 1 June 2015 (2015-06-01), page 89, XP009193143, ISSN: 0390-6078
- C PABA-PRADA: "Determination of the MTD and Encouraging Results in an Ongoing Open-Label Phase 1/2a Study of the Safety and Efficacy of Melflufen and Dexamethasone in Combination for Patients with Relapsed and Relapsed-Refractory Multiple Myeloma (RRMM) | Blood Journal", BLOOD, vol. 124, no. 21, 4 December 2014 (2014-12-04), page 2123, XP055337105,
- D. CHAUHAN ET AL: "In Vitro and In Vivo Antitumor Activity of a Novel Alkylating Agent, Melphalan-Flufenamide, against Multiple Myeloma Cells", CLINICAL CANCER RESEARCH, vol. 19, no. 11, 12 April 2013 (2013-04-12), pages 3019-3031, XP055288158, US ISSN: 1078-0432, DOI: 10.1158/1078-0432.CCR-12-3752
- BERGLUND ÅKE ET AL: "First-in-human, phase I/IIa clinical study of the peptidase potentiated alkylator melflufen administered every three weeks to patients with advanced solid tumor malignancies", INVESTIGATIONAL NEW DRUGS, MARTINUS NIJHOFF PUBLISHERS, BOSTON, US, vol. 33, no. 6, 10 November 2015 (2015-11-10), pages 1232-1241, XP035906340, ISSN: 0167-6997, DOI: 10.1007/S10637-015-0299-2 [retrieved on 2015-11-10]
- VOORHEES PETER M ET AL: "Efficacy of Melflufen, a Peptidase Targeted Therapy, and Dexamethasone in an Ongoing Open-Label Phase 2a Study in Patients with Relapsed and Relapsed Refractory Multiple Myeloma (RRMM) Including an Initial Report on Progression Free Survival", BLOOD, AMERICAN SOCIETY OF HEMATOLOGY, US, vol. 126, no. 23, 1 December 2015 (2015-12-01), XP009193142, ISSN: 0006-4971
- RAY ARGHYA ET AL: "A novel alkylating agent Melflufen induces irreversible DNA damage and cytotoxicity in multiple myeloma cells", BRITISH JOURNAL OF HAEMATOLOGY, vol. 174, no. 3, August 2016 (2016-08), pages 397-409, XP055356365,

## Description

### Field of invention

The present invention relates to a particularly advantageous dosage regimen of melflufen (melphalan flufenamide; L-Melphalanyl-4-fluoro-L-phenylalanine ethyl ester), or a salt thereof, for use in the treatment of multiple myeloma.

### Background of the invention

Multiple myeloma (MM) is a malignant cancer of the differentiated plasma cells. It is characterized by clonal proliferation of plasma cells in the bone marrow and the production of excessive amounts of a monoclonal immunoglobulin (usually of the IgG or IgA type or free urinary light chain [paraprotein, M-protein or M-component]).

MM affects older patients, with a median age at onset of 65 to 70 years and a slight male predominance. MM is the second most common hematologic malignancy and nearly 24,000 patients with myeloma are diagnosed in the United States each year. Patients with MM may experience significant detriment to quality of life, including bone pain, bone fractures, fatigue, anaemia, infections, hypercalcemia, hyperviscosity and renal function compromise (including renal failure). The disease course for MM varies with the disease stage at diagnosis, cytogenetic profile, as well as age and patient comorbidities. The disease is ultimately fatal, with a median survival of approximately 3 to 5 years and a 5-year survival estimated at 44.9% ("Surveillance, Epidemiology, and End Results Program Cancer statistics Stat Fact Sheets: Myeloma." National Cancer Institute;
http://www.seer.cancer.gov/statfacts/html/mulmy.html). However, some patients can live longer than 10 years.

Recent improvements in therapies have significantly prolonged survival, but despite these considerable improvements MM remains incurable and uniformly fatal. Patients presenting with symptomatic active disease receive induction therapy, and potentially consolidation and maintenance therapy. Invariably, relapse occurs following the initial treatment regimen and salvage therapy is needed. Given the inevitable relapses seen in MM patients, new approaches to therapy are needed. Melflufen (also known as melphalan flufenamide and L-Melphalanyl-4-fluoro-L-phenylalanine ethyl ester), is an anti-tumor agent useful in treatment of multiple myeloma. Melflufen is described in WO 01/96367 and WO 2014/065751. The structure of the hydrochloride salt of melflufen is shown in Scheme 1 below:

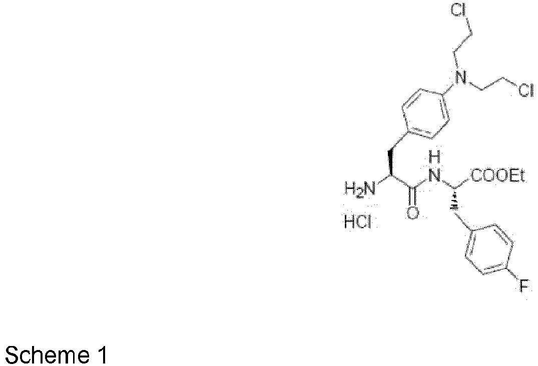

Melflufen is a potent and highly lipophilic alkylating agent and it achieves targeted delivery of alkylating metabolites to tumor cells.

The addition of melflufen to panels of primary cultures of human tumor cells, including MM cells, results in a similar pattern of activity to that of melphalan, but with 50 to 100 fold higher efficacy (Wickstrom, M., et al, Invest New Drugs (2008) Vol 26, pages 195 - 204), which is explained by the 10 to 20 fold higher intracellular concentration (Gullbo, J., et al, J Drug Target, (2003) Vol 11, pages 355-363; Wickstrom, M., et al, Biochem Pharmacol (2010) Vol 79, pages 2381 - 1290). Importantly, *in vitro* studies in MM cell lines resistant to dexamethasone, bortezomib and melphalan have shown cytotoxic activities of melflufen at concentrations similar to those observed in the parenteral, non-resistant cell lines. Potent cytotoxic activity has also been demonstrated *in vitro* in primary MM cells from patients including those relapsing after multiple prior therapies with bortezomib, lenalidomide, and dexamethasone. In efficacy studies conducted in mice and rats carrying different human tumors, including MM, superior antitumor activity of melflufen over equimolar dosage of melphalan was observed at seemingly comparable toxicity (Gullbo, J., et al, Invest New Drugs (2004) Vol 22, pages 411 - 420, Wickstrom, M., et al Mol Cancer Ther (2007) Vol 6, pages 2409-2417, Chauhan, D., et al, Clin Cancer Res (2013) Vol 19, pages 3019 - 3031). Preliminary results of a trial of melflufen in human MM sufferers have been published.

### Summary of the Invention

The present invention provides melflufen (melphalan flufenamide; L-Melphalanyl-4-fluoro-L-phenylalanine ethyl ester), or a salt thereof, for use in the treatment of multiple myeloma, wherein a 37.5 to 42.5 mg dosage of melflufen (excluding the mass of any salt) is administered as a parenteral dosage at an infusion rate of 1.2 to 1.4 mg/min, and wherein the dosage of melflufen, or a salt thereof, is taken on day 1 of a cycle of 28 days.

The dosage regimen of the invention is efficacious whilst at the same time not causing adverse effects to an taken on unacceptable degree. It is postulated that this surprising effect is caused by non-linear pharmacokinetics of the compound. Melflufen is broken down relatively rapidly in the body and the compound and its metabolites become distributed in various locations around the body; the distribution and effects of melflufen and its metabolites appears to be influenced by the rate at which it is infused into the body. It is postulated that the non-linear kinetics enable beneficial effects occur at an infusion rate that is lower than the rate at which unacceptable adverse events are observed.

The present inventors have surprisingly found that the dosage regimen of the invention is particularly effective in the treatment of multiple myeloma, especially relapsed-refractory multiple myeloma.

### Brief Description of the Drawings

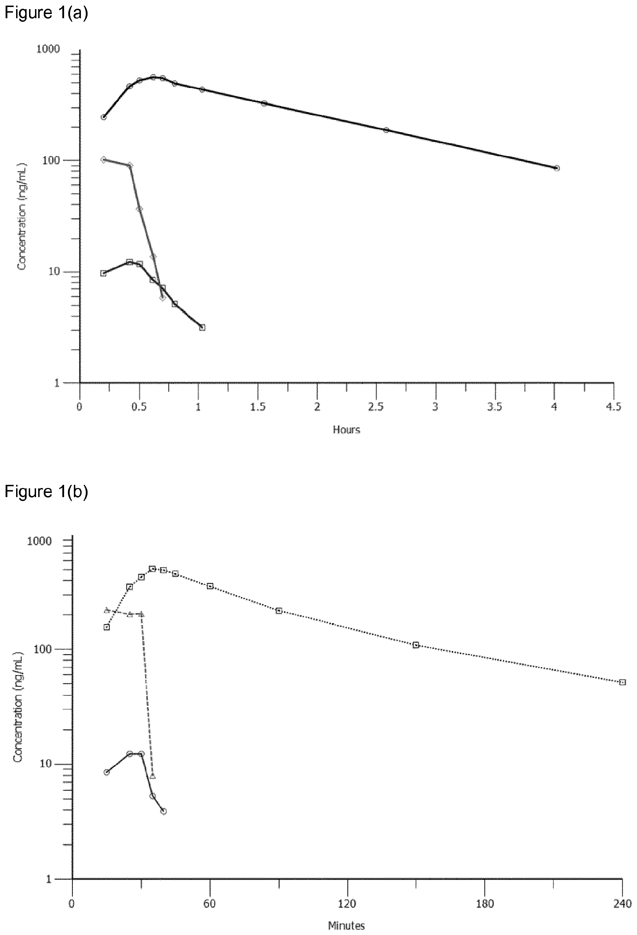

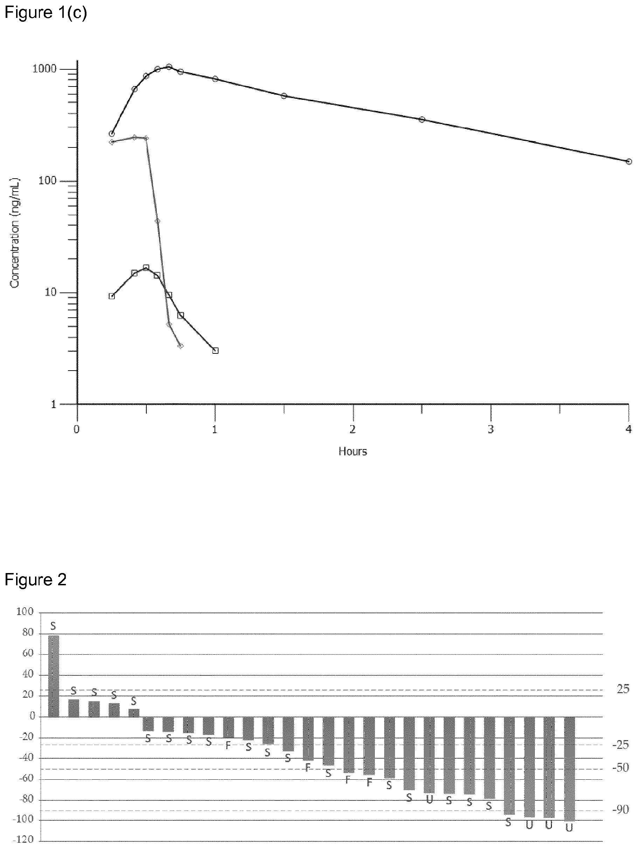

Figures 1(a) and 1(c) show the concentration-time profiles for melflufen (diamonds), melphalan (circles) and des-ethyl-melflufen (squares) after infusion of melflufen over 30 minutes in a patient at the dosage level 25 mg (Figure 1(a)) and 55 mg (Figure 1(c)) of melflufen hydrochloride (excluding the mass of the salt component). Figure 1(b) show the concentration-time profiles for melflufen (triangles), melphalan (squares) and des-ethyl-melflufen (circles) after infusion of melflufen over 30 minutes in a patient at the dosage level 40 mg.
Figure 2 shows the percentage change in paraprotein levels for patients that were evaluable for efficacy in Example 2a (n = 27). The terms S, F and U in Figure 2 refer to how the paraprotein level was measured in each patient: S = serum protein electrophoresis; F = free light chain and U = urine protein electrophoresis.

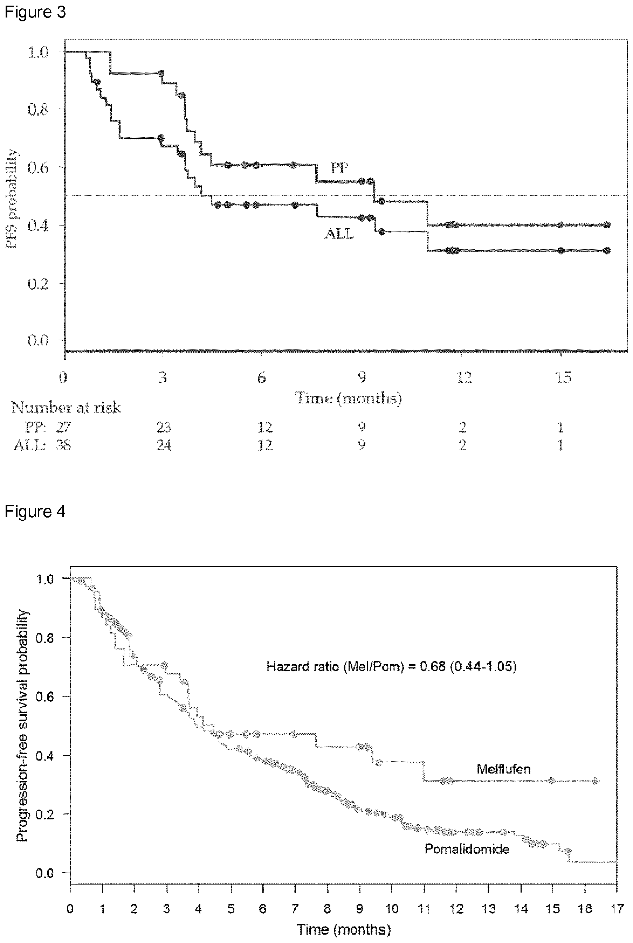

Figure 3 shows a Kaplan-Meier Plot of progression free survival (PFS) for all patients in Example 2a treated with at least one dosage of 40 mg melflufen (as melflufen hydrochloride) as an intravenous dosage over 30 minutes ("ALL") (n=38), and for the efficacy evaluable patients ("PP") of Example 2a, as described below (n = 27).
Figure 4 shows a Kaplan-Meier Plot of progression free survival (PFS) for all patients in Example 2a treated with at least one dosage of 40 mg melflufen (as melflufen hydrochloride) as an intravenous dosage over 30 minutes, and the PFS for the approved drug pomalidomide (San Miguel, J., et al., Lancet Oncol, (2013), Vol 14, pages 1055-1066).

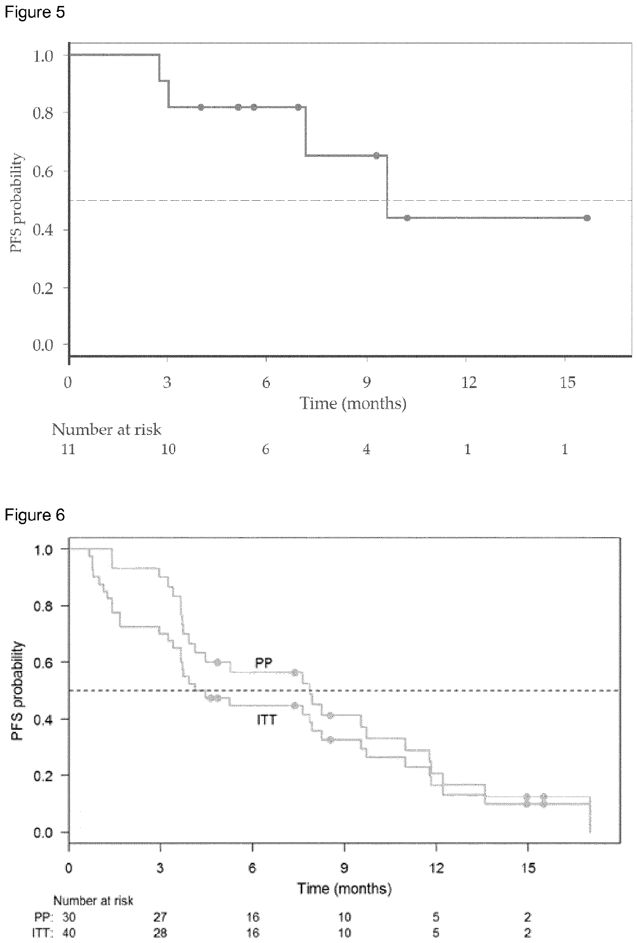

Figure 5 shows a Kaplan-Meier Plot of duration of response (DOR) in the 11 patients who responded to melflufen treatment in Example 2a.
Figure 6 shows a Kaplan-Meier Plot of progression free survival (PFS) for all patients in Example 2b treated with at least one dosage of 40 mg melflufen (as melflufen hydrochloride) as an intravenous dosage over 30 minutes ("ITT") (n=40), and for the efficacy evaluable patients ("PP") of Example 2b, as described below (n = 30).

### Detailed Description of Invention

The present invention finds utility in the treatment of multiple myeloma, especially in the treatment of relapsed-refractory multiple myeloma. The present invention provides melflufen, or a salt thereof, for use in the treatment of multiple myeloma, wherein a 37.5 to 42.5 mg dosage of melflufen (excluding the mass of any salt) is administered as a parenteral dosage at an infusion rate of 1.2 to 1.4 mg/min, and wherein the dosage of melflufen, or a salt thereof, is taken on day 1 of a cycle of 28 days.

Disclosed herein is melflufen, or a salt thereof, for use in the treatment of multiple myeloma, wherein a dosage of melflufen (excluding the mass of any salt) is administered as a parenteral dosage at an infusion rate of 1.0 to 1.8 mg/min.

Also disclosed herein is melflufen, or a salt thereof, for use in the treatment of multiple myeloma, wherein a dosage of melflufen (excluding the mass of any salt) of 37.5 to 42.5 mg is administered as a parenteral dosage over 25 - 35 minutes.

The present invention is especially useful for the treatment of refractory, relapsed, and/or relapsed refractory multiple myeloma. The invention finds utility in the treatment of a mammal, especially a human, having multiple myeloma.

Surprising benefits for a dosage of melflufen (excluding the mass of any salt) of 37.5 to 42.5 mg administered as a parenteral dosage over 25 - 35 minutes have been found by the present inventors:
The clinical studies described in Example 1 below show that after intravenous infusion, melflufen very rapidly disappears from plasma with no signs of redistribution back to the plasma, indicating that a complete metabolism occurs predominantly outside the plasma compartment.

In contrast to other alkylating agents that are hydrophilic, the lipophilicity of melflufen leads to rapid and extensive distribution into tissues and cells. Inside cells, melflufen may directly bind DNA or is readily metabolized by intracellular peptidases into the antitumor compound melphalan or by esterases into des-ethylmelflufen, which also has alkylating properties. Due to the high activity of peptidases and esterases in human tumor cells, the formation of melflufen's metabolites is rapid in these cells with subsequent inflow of more melflufen (Gullbo, J., et al, J Drug Targer, (2003) Vol 11, pages 355-363; Wickstrom, M., et al, Biochem Pharmacol (2010) Vol 79, pages 2381 - 1290). Since des-ethylmelflufen and melphalan are relatively hydrophilic, there is a possibility for intracellular trapping of these agents. In MM cells *in vitro,* melflufen gives at least a 20-fold higher intracellular exposure (as AUC) of alkylating agents compared to that observed after an equimolar dosage of melphalan (Chauhan, D, et al, Clin Cancer Res (2013) Vol 19, pages 3019-3031). This can be explained by a more efficient transport of melflufen into these cells, an efficient conversion into other molecules (i.e. melphalan and des-ethyl-melflufen) inside the cells and a less rapid disappearance of these molecules from the cells.

The above-mentioned behaviours and distribution properties of melflufen are now supported by clinical pharmacokinetic data in humans.

The present inventors have found that following administration of melflufen hydrochloride, melphalan is found in plasma with a peak concentration at 5 to 10 minutes after the end of melflufen infusion (see Examples section 2.1, below). The dosing of melflufen at an infusion rate of 1.0 to 1.8 mg/min (for example as an infusion of 40 mg over 30 minutes) is particularly compatible with these kinetics: it enables delivery of an effective dosage of melphalan to the necessary compartments without systemic levels of melphalan being so high as to cause adverse effects. The total melphalan plasma exposure assessed as AUC after melflufen administration is similar to historical data on exposure after melphalan administration (Mougenot, P., et al, Cancer Chemother Pharmacol (2004) Vol 53, pages 503-512; Nath, C. E., et al, Br J Clin Pharmacol (2010) Vol 69, pages 484-497). However, the intracellular concentration of melphalan in tumor cells is likely to be considerably higher, as discussed above.

The present inventors have surprisingly found that when administering melflufen, the infusion rate is very important, and will effect both the safety and efficacy of the dosage (see Example 1, and Figures 1(a) to (c)). When the infusion rate is less than 1.8 (for example a dose of melflufen of 25 or 40 mg administered over 30 minutes) Cₘₐₓ and AUC_{inf} are significantly lower than would be expected compared to an infusion rate of over 1.8 (for example a dose of 55 mg over 30 minutes). It can be concluded from this data that is there is a non-linear relationship between dose and safety, and when the infusion rate is kept lower than 1.8 mg/min, the reduction in the risk of toxicity and side effects is significant.

The present inventors have further found that the safety profile of melflufen when dosed at an infusion rate of 1.0 to 1.8 mg/min (for example at 40 mg over 30 minutes) is good. It is similar to that for other alkylators, with neutropenia and thrombocytopenia as the most common adverse events. In patients treated with 40 mg of melflufen (as melflufen hydrochloride) over 30 minutes in all treatment cycles, the incidences of Grade 3 and Grade 4 events of neutropenia and thrombocytopenia were at an acceptable level. When a higher dosage was given, the incidences of Grade 3 and Grade 4 events of neutropenia and thrombocytopenia were significantly higher, as were incidences of Grade 3 and Grade 4 infections and infestations (see Table 7 and Table 13, below).

In summary, an infusion rate of 1.0 to 1.8 mg/min of melflufen (for example a dosage of 35 to 45 mg of melflufen administered over 25 to 35 minutes) can be used in MM patients without the risk of redistribution of melflufen back to the plasma, which could increase toxicity and side effects, whilst providing higher intracellular levels of melphalan compared to administration of melphalan itself. The safety profile supports this, and shows the dosage of melflufen of the present invention does not have greater risk of adverse effects than known, approved alkylators.

Furthermore, the benefit of the present dosage regime to patients having MM is surprisingly significant. The data disclosed herein show that the claimed dosage regime is an especially effective treatment of MM.

Analysis of data from Example 2 was performed (see Examples section 3.1 and 4.1, below). Looking at the results from Example 2b, which is the data with the latest data cut-off point), of the 30 patients with advanced relapsed and relapsed-refractory MM treated with ≥ 2 cycles of 40 mg melflufen (as melflufen hydrochloride) administered as an intravenous dosage over 30 minutes in combination with dexamethasone (with median of 4 prior lines of therapy, including IMiD, PI and melphalan in all but two patients), 19 patients (63%) reported a best response of minimal response (MR) or better; and 12 patients (40%) reported partial response (PR) or better. The median progression free survival (PFS) in Example 2b was 7.9 months based on events in the efficacy evaluable population (n=30). That is an increase in PFS of around 4 months compared to the recently approved drugs pomalidomide + dexamethasone (PFS in Phase II = 4.2 months: Richardson, P., et al, Blood (2014) Vol 123, Pages 1826 - 1832; PFS in Phase III = 4.0: San Miguel, J., et al., Lancet Oncol, (2013), Vol 14, pages 1055-1066; and PFS in Phase III based on the assessment by the Independent Review Adjudication Committee (IRAC) review at the final PFS analysis = 3.6 months (FDA Pomalyst label (2015) http://www.accessdata.fda.gov/drugsatfda_docs/label/2015/204026s005s006s008lbI.pdf) and carfilzomib (PFS in Phase II = 3.7 months: Siegel, D. S., et al, Blood (2012) Vol 120, pages 2817 - 2824).

Analysis of data from Example 2a, which is the data having cut-off point (a), the hazard ratio of melflufen treatment compared to pomalidomide treatment is 0.68 (0.44 - 1.05). Figure 4, the Kaplan-Meyer plots for melflufen and pomalidomide, clearly shows that especially in the time after 5 months, melflufen treatment leads to slower progression of MM.

Melflufen administered by the regimen of the present invention therefore offers significant improvements in survival in a disease which presently has no cure. The dosage regime of the present invention therefore offers patients valuable time not provided by currently available drugs for MM.

Example 2 and the clinical studies mentioned above for which the PFS were reported include a large proportion of refractory MM patients; the results from Example 2 compare favourably with the other studies mentioned above. At the Example 2b data cut-off point, refractory status was available for 29 of the 30 patients in Example 2a. Of these 29 patients, 24 (83%) were IMiD-refractory, 19 (66%) were PI-refractory and 15 (52%) were alkylator refractory. Seventeen (17) of 29 patients (59%) were double-refractory (IMiDs and PIs) and 9 (31%) were double- and alkylator-refractory. Twenty-three (23) of 29 patients (79%) were refractory to last line of treatment. The median number of prior therapies was 4 (range 1 to 13) in all 40 patients. The available data show that responses to melflufen combined with weekly 40 mg dexamethasone are rapid and durable. Of the 30 patients evaluated, 19 patients (63%) have reported a best response of MR or better and 12 patients (40%) have reported a PR or better.

As mentioned above in Example 2b, 15 of the 29 patients (52%) were refractory to an alkylator. 8 of the 15 alkalytor refractory patients achieved at least a PR. In fact, 8 of the 11 patients who achieved at least a PR were alkalytor refractory. This is significant because it demonstrates activity for melflufen when administered by the regiment of the present invention that is independent of drug class switch, which is often used as an effective strategy in later lines of therapy. Finally, the present inventors have found that, unlike many active ingredients where the amount which is required to achieve a therapeutic effect will vary with the subject under treatment, including the type, age, weight, sex, and medical condition of the subject and the renal and hepatic function of the subject, as well as the severity of the multiple myeloma, that is not the case for melflufen. Very surprisingly, the dosage regime of the present invention is suitable for all subjects, and does not need to be modified in view of the detailed characteristics of the patient. As such, the present invention provides a simple dosage regimen that is effective in all patients having MM without individual dose titration being necessary. The inventors have also found that melflufen does not appear to have any effect on renal function, and thus in patients with poor renal function, the dosage of melflufen would not need to be reduced. Doses of melphalan are generally reduced in patients having poor renal function.

In summary, the clinical results described herein support that melflufen administered according to the dosage regime of the invention provides targeted delivery of alkylating metabolites to tumor cells (such as MM cells) and thereby exerts a higher anti-tumor activity compared with equimolar administration of melphalan and with a similar safety profile. Furthermore, in Example 2b (i.e. the Example 2 data with the latest cut-off date) an especially good median PFS of 7.9 months (based on events in the efficacy evaluable population) is achieved when patients are treated following the dosage regime of the invention. The efficacy is persistent across MM populations including RRMM patients who are double-refractory and refractory to alkylators.

### Melflufen and salts thereof

Melflufen, and salts thereof, especially the hydrochloride salt thereof, are known from, for example, WO 01/96367 and WO 2014/065751.

For the avoidance of doubt, in this document, when the term "melflufen" is used, it includes salt(s) thereof, unless stated otherwise.

Also for the avoidance of doubt, when referred to in this document, the mass of melflufen is the mass of the melflufen molecule excluding the mass of any salt component unless explicitly stated otherwise.

Salts of melflufen which are suitable for use in the present invention are those wherein a counterion is pharmaceutically acceptable. Suitable salts include those formed with organic or inorganic acids. In particular, suitable salts formed with acids according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as (C₁-C₄)-alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxalic, oxaloacetic, methanesulfonic, ethanesulfonic, p-toluenesulfonic, formic, benzoic, malonic, naphthalene-2-sulfonic, benzenesulfonic, isethionic, ascorbic, malic, phthalic, aspartic, and glutamic acids, lysine and arginine.

Preferred salts of melflufen include acid addition salts such as those formed from hydrochloric, hydrobromic, acetic, p-toluenesulfonic, tartaric, sulphuric, succinic, phosphoric, oxalic, nitric, methanesulfonic, malic, maleic and citric acid. More preferably, the salt of melflufen according to the present invention is the hydrochloride salt (i.e. the addition salt formed from hydrochloric acid).

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Before it is made up in solution, the melflufen, or a salt thereof, for use in the present invention may be in the form of a solvate.

### Dosages and formulations

Disclosed herein is melflufen, or a salt thereof, for use in the treatment of multiple myeloma, wherein a dosage of melflufen (excluding the mass of any salt) is administeted as a parenteral dosage at an infusion rate of around 1.0 to 1.8 mg/min.

Disclosed herein is melflufen, or a salt thereof, for use in the treatment of multiple myeloma, wherein a dosage of melflufen (excluding the mass of the salt) of around 37.5 to 42.5 mg (preferably around 40 mg) is administered as a parenteral dosage over around 25 - 35 minutes (preferably around 30 minutes).

The infusion rate of melflufen for use in the treatment of multiple myeloma is 1.2 to 1.4 mg/min, for example 1.2, 1.3 or 1.4 mg/min. Preferably the infusion rate of melflufen for use in the present invention is 1.3 mg/min for example 1.33 mg/min.

The maximum total dosage of melflufen, or salt thereof, (excluding the mass of any salt) is 42.5 mg. The minimum total dosage of melflufen, or salt thereof, (excluding the mass of any salt) is 37.5 mg.

Preferably, the maximum length of the infusion is 35 minutes, more preferably 33 minutes. Preferably, the minimum total length of the infusion is 25 minutes, and more preferably 27 minutes.

As regards the dosage of melflufen for use in the present invention, when a mass of melflufen or a salt thereof is referred to, that is the mass when no salt component is included in the calculation of the dosage mass of the melflufen. The molecular weight of salt-free melflufen is 498.42 g/mol. For a dosage of a salt of melflufen, the actual dosage mass administered to the patient must take into account the mass of the salt. This is routine for the person skilled in the art.

For example, when the melflufen is in the form of its hydrochloride (HCl) salt it has a molecular weight of 534.88 g/mol. For a dosage of melflufen of 35 to 40 mg, the equivalent dosage of melflufen hydrochloride will be approximately 37.6 to 48.3 mg.

The present invention is also directed to melflufen hydrochloride, for use in the treatment of multiple myeloma, wherein a dosage of melflufen (including the mass of the salt) is administered as a parenteral dosage at an infusion rate of around preferably 1.4 mg/min.

Also disclosed herein is melflufen hydrochloride for use in the treatment of multiple myeloma, wherein a dosage of melflufen hydrochloride (including the mass of the salt) of around 37.6 to 48.3 mg (preferably 40 to 45 mg, and more preferably 42.9 mg) is administered as a parenteral dosage over 25 - 35 minutes (preferably 30 minutes).

The melflufen, or salt thereof, of the present invention is administered as a dosage of 37.5 to 42.5 mg (for example 37.5, 38.0, 38.5, 39.0, 39.5, 40.0, 40.5, 41.0, 41.5, 42.0 or 42.5 mg), more preferably 38.0 to 42.0 mg; and most preferably 39.0 to 41.0 mg (for example 39.0, 39.5, 40.0,40.5 or 41.0 mg more preferably 39.5, 40.0 or 40.5 mg and most preferably 400 mg).

In embodiments where melflufen is in the form of its HCl salt, a dosage of 37.6 to 48.3 mg, preferably 39.0 to 47.0 mg, more preferably 41.0 to 45.0 mg, more preferably 42.5 to 43.5 mg and most preferably 42.9 mg, of melflufen hydrochloride (including the mass of the salt component), is administered as a parenteral dosage over 25 - 35 minutes.

Preferably the melflufen, or salt thereof, of the present invention is administered over 26 to 34 minutes, more preferably over 27 to 33 minutes, even more preferably over 28 to 32 minutes, even more preferably over 29 to 31 minutes, and most preferably over 30 minutes.

The dosage regime of the present invention is administered as a parenteral dosage, and thus the dosage of melflufen must be in the form of a liquid, for example a solution or suspension comprising the melflufen.

The melflufen, or salt thereof, of the present invention is taken as part of a treatment cycle. In a cycle, the melflufen may be administered on day 1 of the cycle, wherein the cycle lasts X days, with no further melflufen administered for the next X-1 days. X is 28 days. In the dosage regime of the present invention, melfluten, or a salt thereof, is administeried on day 1 of a 28 day cycle followed by 27 days of rest with no further melflufen being administered during that time.

The cycle may be repeated one or several times depending on the category, class or stage of the MM. For example the cycle may be repeated from 1 to 15 times, for example from 2 to 12 times, for example 2 to 7 times, for example 2, 3, 4, 5, 6 or times. The cycle may be is repeated, 3, 4 or 5 times.

An ordinarily skilled physician or clinician can readily determine the number of cycles of melflufen, or a salt thereof, required to prevent, counter or arrest the progress of the multiple myeloma.

The melflufen for use in the present invention may be provided as a unit dosage. Preferred unit dosage formulations for use in the present invention are those containing a requisite dosage of melflufen, as hereinbefore recited. Preferably the unit dosage is 40 mg (for example 40.0 mg).

The melflufen for use in the present invention may be provided as a divided dosage (i.e. such that when multiple divided dosages are aggregated, a unit dosage of melflufen is arrived at). Preferred divided dosages for use in the present invention are those containing an appropriate fraction of a dosage of the melflufen hereinbefore recited. A plurality (two or more [for example two, three or four; preferably two]) of divided dosages of melflufen can be provided to arrive at a unit dosage (i.e. a requisite dosage of melflufen as hereinbefore recited). The plurality of divided dosages provided to make a unit dosage may be the same divided dosage (for example 2 x 20 mg dosages can be provided to arrive at a 40 mg unit dosage), or may be different divided dosages (for example 1 x 20 mg dosage and 2 x 10 mg dosage can be provided to arrive at a 40 mg unit dosage).

A divided dosage of melflufen, or a salt thereof (excluding the weight of any salt), may be, 1 to 35 mg: for example 1 mg, 5 mg, 10 mg, 12 mg, 12.5 mg, 15 mg, 17.5 mg, 18 mg, 19 mg, 20 mg, 22.5 mg, 25 mg, 27.5 mg, 30 mg, 32.5 mg or 35 mg. Preferably a divided dosage is from 10 to 25 mg.

Where the melflufen is in the form of its HCl salt, a divided dosage of melflufen hydrochloride (including the mass of the salt) may be from 1 to 35 mg: for example, 1 mg, 1.45 mg, 5 mg, 10 mg, 12 mg, 12.5 mg, 12.9, 15 mg, 16 mg, 17 mg, 17.5 mg, 17.9 mg, 18 mg, 19 mg, 19.5 mg, 20 mg, 21.45 mg, 22.5 mg, 22.9 mg, 25 mg, 27.5 mg, 22.9 mg, 30 mg, 32.5 mg or 35 mg. Preferably a divided dosage is from 10 to 25 mg. Preferably the divided dosage is 21.45 mg (preferably 2 x 21.45 mg dosages are provided to arrive at a 42.9 mg unit dosage of melflufen hydrochloride).

In the dosage regimen of the present invention, the dosage of melflufen, or a salt thereof, is administered as a parenteral dosage. As such, pharmaceutical formulations useful according to the invention are those suitable for parenteral administration.

Parenteral administration includes intravenous (into a vein) (bolus or infusion), intraarterial (into an artery), intraosseous infusion (into the bone marrow), intra-muscular (into muscle), intradermal (into the dermis), and subcutaneous (under the skin) administration. Preferably, the dosage of the present invention is administered intravenously or intra-arterially, and more preferably by intravenous infusion. As such, pharmaceutical formulations especially useful for the present invention are those suitable for intravenous administration, and more especially intravenous infusion. The rate of infusion is preferably a constant rate infusion.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. Preferably the formulations may be presented in unit dosage or divided dosage containers, for example sealed ampoules and vials. The formulation may be stored in a freeze-dried (lyophilised) condition requiring only the addition of the sterile liquid carrier, for example saline, a physiologically acceptable solution or water-for-injection, immediately prior to use.

Extemporaneous injection and infusion solutions and suspensions may be prepared from sterile powders, granules or other dry composition. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Preferably, the dosage of melflufen is administered as a pharmaceutical solution. Preferably, the dosage of melflufen is administered as a pharmaceutical solution having a volume of 1 to 1500 ml; preferably from 10 to 1000 ml, more preferably from 100 to 600 ml, more preferably from 150 to 500 ml, more preferably from 200 to 450 ml, and even most preferably from 250 to 400 ml (for example 250, 260, 270, 275, 280, 290, 300, 310, 320, 325, 330, 340, 350, 360, 370, 375, 380, 390 or 400 ml) and most preferably 275 to 400 ml (for example 290, 300, 320, 325, 330, 340, 350, 360, 370, 375 or 400 ml). It is especially preferred that the dosage of melflufen is administered as a pharmaceutical solution having a volume 290 to 370 ml, for example 290, 300, 330, 350 or 370 ml, preferably 300 to 350 ml, for example 330 ml.

Preferably, the dosage of melflufen is administered as a pharmaceutical solution comprising a physiologically acceptable solution, such as a glucose solution. The wording a "physiologically acceptable solution" as used herein, may be an aqueous solution, such as a NaCl solution (such as about 0.9 wt % NaCl) or a glucose solution (such as about 4.5-5.5 wt % glucose, e.g. about 5 wt %), or another physiologically acceptable solution. Any such solution may optionally be buffered. Preferably a physiologically acceptable solution of melflufen for use in the present invention is a glucose solution, preferably a 4.5-5.5 wt % glucose solution, and most preferably a 5 wt % glucose solution.

A pharmaceutical solution comprising melflufen, or a salt thereof, (for example lyophilized melflufen, or a salt thereof) and a physiologically acceptable solution for direct administration to a subject, generally comprises melflufen, or a salt thereof, at a concentration of about 1.2 mg/mL or less, preferably 1.0 mg/mL or less, such as about 0.2 mg/mL. For example, a pharmaceutical solution comprising melflufen, or a salt thereof, for use in the present invention may have a concentration of 0.01 mg/mL to 1.2 mg./mL, preferably 0.05 mg/mL to 1.0 mg/mL, more preferably 0.01 mg/mL to 0.5 mg/mL, for example 0.1 or 0.2 mg/mL.

The pharmaceutical solution may comprise melflufen, or a salt thereof, in a concentration of up to about 4 mg/ml, which may be diluted by the mixture with further physiologically acceptable solution (for example to a concentration of about 0.001 mg/mL to 1.2 mg/ml, such as about 0.2 mg/ml) before administration to a patient.

A pharmaceutical composition of melflufen, or a salt thereof, may be provided that can be made into a pharmaceutical solution by addition of a sterile liquid carrier, for example a physiologically acceptable solution. A pharmaceutical composition of melflufen, or a salt thereof, may be provided in a vial, so that a solution of concentration 0.001 mg/mL to 4 mg/mL, preferably from 0.05 to 2.5 mg/mL, more preferably from 0.1 to 1.2 mg/mL, and even more preferably 0.3 to 0.6 mg/mL (for example 0.3, 0.4, 0.5 or 0.6 mg/mL) can be produced when a sterile liquid carrier, for example a physiologically acceptable solution, is added to the vial. That solution may be further diluted with further sterile liquid carrier, preferably further physiologically acceptable solution, before administration to a patient (for example to a concentration of about 0.001 mg/mL to 1.2 mg/ml, such as about 0.1 or about 0.2 mg/ml).

A pharmaceutical composition of melflufen may be provided in a 1 to 200 ml vial, preferably a 10 to 100 ml vial, more preferably a 30 to 60 ml vial, and most preferably a 50 ml vial, so that a solution of concentration 0.1 mg/mL to 4 mg/mL, preferably from 0.2 to 2.5 mg/mL, more preferably from 0.2 to 1.2 mg/mL and even more preferably 0.3 to 0.6 mg/mL (for example 0.3, 0.4, 0.5 or 0.6 mg/mL) may be produced when physiologically acceptable solution is added to the vial. That solution may be further diluted as described above before administration to a patient.

Such a vial may comprise a unit dosage of melflufen, as described above, or a divided dosage of melflufen as described above, which when multiple divided dosages are provided, a unit dosage of melflufen is arrived at (e.g. for a unit dosage of 40 mg of melflufen, two vials (e.g. 50 ml vials) each having a divided dosage of 20 mg may be provided to achieve the unit dosage of 40 mg; for example for a unit dosage of 42.9 mg of melflufen hydrochloride, two vials each having a divided dosage of 21.45 mg may be provided to achieve the 42.9 mg unit dosage).

Preferably, the melflufen, or salt thereof, for use in the present invention comprises a lyophilized pharmaceutical preparation of a melflufen or a salt thereof. The term "lyophilized pharmaceutical preparation of a melflufen or a salt thereof" is understood to mean that the melflufen or a salt thereof is free-dried ("Lyophilization", "lyophilized" etc. may in the present context be used interchangeably with "freeze-drying", "freeze-dried" etc.). A lyophilized pharmaceutical preparation of melflufen or a salt thereof as described herein may be a white, fluffy powder in contrast to a non-lyophilized melflufen or a pharmaceutically acceptable salt thereof, which is typically in the form of a dense, slightly yellowish powder.

A lyophilized pharmaceutical preparation of melflufen, or a salt thereof, for use in the present invention may comprise sucrose. The inclusion of sucrose provides lyophilized preparation that is stable as such, and water-soluble, without the presence of an organic solvent, at a sufficient rate compared to the degradation rate, and is thereby useful in therapy and does not have toxicity brought about by the organic solvent. Due to the increased solubility and/or rate of dissolution of melflufen, or a salt thereof, after lyophilization in the presence of sucrose, it is possible to prepare a dissolved melflufen, or a salt thereof, solution, such as a pharmaceutical composition comprising melflufen, or a salt thereof, which has a usefully high concentration of melflufen and which is substantially free from organic solvents. Preparation of a lyophilized pharmaceutical preparation, a lyophilized pharmaceutical composition, and a kit for making such compositions, of melflufen or a salt thereof, is described in detail in WO 2012/146625 and WO 2014/065741.

A pharmaceutical formulation of melflufen, or a salt thereof, for use in the present invention may comprise a lyophilized pharmaceutical preparation comprising melflufen, or a salt thereof. Where the formulation is a pharmaceutical solution, it may be prepared from a lyophilized pharmaceutical preparation comprising melflufen, or a salt thereof, and further comprise a physiologically acceptable solvent, such as a glucose solution.

It should be understood that in addition to the ingredients particularly mentioned above, the formulations of this invention may include other agents conventional in the art having regard to the type of formulation in question.

Whilst melflufen, or a salt thereof, may be used as the sole active ingredient in the present invention, it is also possible for it to be used in combination with one or more further therapeutic agent(s), and the use of such combinations provides one preferred embodiment of the invention. Such further therapeutic agents may be agents useful in the treatment of multiple myeloma, or other pharmaceutically active materials. Such agents are known in the art. Examples of further therapeutic agents for use in the present invention include steroids (prednisone and dexamethasone), IMiDs (thalidomide, lenalidomide and pomalidomide), PIs (bortezomib, carfilzomib and ixazomib), histone deacetylase (HDAC) inhibitors (panobinostat), conventional chemotherapy (alkylators (e.g. melphalan, cyclophosphamide, bendamustine), doxorubicin), anti-CD38 antibodies (daratumumab) and anti-SLAMF7 antibodies (elotuzumab); for example steroids (prednisone and dexamethasone), IMiDs (thalidomide, lenalidomide and pomalidomide), PIs (bortezomib and carfilzomib), histone deacetylase (HDAC) inhibitors (panobinostat) and conventional chemotherapy (alkylators (e.g. melphalan, cyclophosphamide) and doxorubicin).

Also disclosed herein is melflufen, or a salt thereof, together with one or more further therapeutic agent(s) for use in the treatment of multiple myeloma, wherein a dosage of melflufen is administered at a rate of 1.2 to 1.4 mg/min. For example a dosage of 37.5 to 42.5 mg,(more preferably 39 to 41 mg and most preferably 40 mg) is administered as a parenteral dosage over 25 - 35 minutes (preferably over 30 minutes). Preferably the further therapeutic agent is dexamethasone.

Also disclosed herein is melflufen hydrochloride, together with one or more further therapeutic agent(s), for use in the treatment of multiple myeloma, wherein a dosage of melflufen hydrochloride (including the mass of the salt) is administered at a rate of 1.1 to 1.9 mg/min. For example as dosage of melflufen hydrochloride (including the mass of the salt) of 37.6 to 48.3 mg (preferably 40 to 45 mg, more preferably 42.9 mg), is administered as a parenteral dosage over 25 - 35 minutes (preferably over 30 minutes). Preferably the further therapeutic agent is dexamethasone.

When used in a combination, the precise dosage of the other pharmaceutically active material may vary with the dosing schedule, the potency of the particular agent chosen, the age, size, sex and condition of the subject (typically a mammal, for example a human), the nature and severity of the melanoma, and other relevant medical and physical factors.

The above therapeutic agents, when employed in combination with melflufen or a salt thereof, may be used, for example, in those amounts indicated in the Physicians' Desk Reference (PDR) or as otherwise determined by one of ordinary skill in the art.

Where the further therapeutic agent is dexamethasone, preferably the dosage is from 1 mg to 200 mg, preferably 5 mg to 100 mg, more preferably 10 mg to 80 mg, and most preferably 20 mg to 60mg, for example 40 mg.

The one or more further therapeutic agent(s) may be used simultaneously, sequentially or separately with/from the administration of the dosage of the melflufen, or salt thereof. The individual components of such combinations can be administered separately at different times during the course of therapy or concurrently in divided or single combination forms.

Where the further therapeutic agent is dexamethasone, preferably the dexamethasone is administered on the same day and simultaneously, sequentially or separately from the administration of the melflufen, or salt thereof. More preferably it is administered separately from and on the same day as the melflufen, or salt thereof.

For example, when the melflufen, or a salt thereof, for use in the present invention is taken as part of a treatment cycle (for example melflufen, or a salt thereof is administered on day 1 of a cycle lasting X days, with no further melflufen taken for the next X-1 days), the dexamethasone may be administered simultaneously, sequentially or separately on the same day as the melflufen is administered (i.e. on day 1). X is 28 days.

In one preferred embodiment of the invention dexamethasone is administered on day 1 in a treatment cycle. More preferably dexamethasone is also administered weekly during such a treatment cycle, for example administered on days 1, 8, 15 and 22 of a 28 day cycle.

In another preferred embodiment, melflufen, or a salt thereof is administered, according to the present invention, on day 1 of a 28 day cycle, and dexamethasone is administered simultaneously, sequentially or separately on day 1 on the cycle, followed by 27 days of rest with no further melflufen being administered during that time. Preferably, the dexamethasone is administered separately from the melflufen, or salt thereof, on day 1. Preferably the dexamethasone is administered orally or intravenously.

In another preferred embodiment, when melflufen, or a salt thereof, for use in the present invention taken as part of a cycle (e.g. melflufen is administered on day 1 of a cycle lasting X days, with no further melflufen taken for the next X-1 days), the dexamethasone is administered simultaneously, sequentially or separately on the same day as the melflufen is administered (i.e. on day 1), and weekly thereafter during the cycle. For example dexamethasone is administered on day 1, 8, 15, 22, 29 etc. depending on the length of the cycle. X is 28 days.

In such an embodiment, melflufen, or a salt thereof is administered, according to the present invention, on day 1 of a 28 day cycle, followed by 27 days of rest with no further melflufen being administered during that time, and dexamethasone is administered simultaneously, sequentially or separately on day 1 on the cycle and on days 8, 15 and 22 of the 28 day cycle. Preferably, the dexamethasone is administered separately to the melflufen, or salt thereof, on day 1 as an oral dosage or an intravenous dosage (preferably an oral dosage). The later dosage of dexamethasone may be oral dosages or intravenous dosages (preferably the later dosages are oral dosages).

### Multiple Myeloma

The dosage regime of the present invention is useful for the treatment of cancer, and in particular multiple myeloma. There are several categories of multiple myeloma, including monoclonal gammopathy of undetermined significance (MGUS), asymptomatic myeloma (further subdivided into smoldering myeloma or indolent myeloma), and symptomatic myeloma. Multiple myeloma may be classed as primary, refractory, relapsed and refractory-relapsed.

Relapsed multiple myeloma (also known as recurrent myeloma) can be defined as multiple myeloma that recurs on or within 60 days of last dosage of treatment.

Refractory multiple myeloma can be defined as multiple myeloma that is not responsive to treatment. Refractory myeloma may occur in patients who never see a response from their treatment therapies or it may occur in patients who do initially respond to treatment, but do not respond to treatment after relapse.

Refractory-relapsed multiple myeloma (RRMM) is a specific sub-type of refractory multiple myeloma, and can be defined as multiple myeloma that initially responds to treatment, but does not respond to treatment after relapse.

There are currently 7 classes of approved drugs available for the treatment of MM, namely steroids (e.g. prednisone and dexamethasone), IMiDs (e.g. thalidomide, lenalidomide and pomalidomide), PIs (e.g. bortezomib, carfilzomib, and ixazomib), histone deacetylase (HDAC) inhibitors (e.g. panobinostat),conventional chemotherapy (e.g. melphalan, cyclophosphamide, doxorubicin, bendamustine), anti-CD38 antibodies (daratumumab) and anti-SLAMF7 antibodies (elotuzumab).

Patients presenting with symptomatic active MM receive primary induction therapy. Those under the age of approximately 65 and in otherwise good health are also considered for consolidation therapy with autologous stem cell transplantation to enhance remission duration (Moreau, P., et al, J Clin Oncol (2011), Vol 29, pages 1898-1906; Rosinol, L., et al, Expert Rev Hematol (2014) Vol 7, pages 43-53.). The type of induction therapy will vary greatly depending on age, disease status and presence of other comorbidities. The NCCN Guidelines for Multiple Myeloma (NCCN (2014). "NCCN Guidelines for Patients." National Comprehensive Cancer Network; http://www.nccn.org/patients/guidelines/myeloma/index.html) provide a list of regimens recommended as primary therapy for transplant eligible and non-transplant eligible patients. Regimens including bortezomib and lenalidomide are most often used as primary therapy; these agents are often combined with an alkylator for non-transplant candidates. There are several treatment regimens recommended for patients ineligible for standard stem-cell transplantation in the consensus statement by the International Myeloma Working Group 2014 (Palumbo, A., et al, J Clin Oncol (2014) Vol 32, pages 587-600). Invariably, relapse occurs following each of these agents and salvage therapy is needed.

Refractory multiple myeloma (and/or RRMM) may be refractory to at least one drug from a class of drugs selected from protease inhibitors (Pis), immunomodulatory drugs (IMiDs) or alkylators. Some refractory multiple myeloma (and/or RRMM) will be refractory to one or more (for example 1, 2, 3, 4 or 5 or more) drug from two of more classes of drugs selected from protease inhibitors (Pis), immunomodulatory drugs (IMiDs) or alkylators). Refractory multiple myeloma (and/or RRMM) may even be refractory to two or more drugs from two or more classes of drugs selected from protease inhibitors (Pis), immunomodulatory drugs (IMiDs) or alkylators).

The choice of treatment for any individual with disease relapse will depend on a number of variables, including response and duration to initial chemotherapy, comorbidities, marrow reserve and whether the patient experiences an indolent or aggressive relapse. The selection of treatment in RRMM is especially challenging. Multiple therapies and combinations of some of the approved drugs mentioned above are available for the treatment of RRMM. In general, myeloma patients will receive an average of 4 to 8 different regiments during their lifespan. However, despite the availability of effective therapies, the optimal combinations and sequencing of these agents with other therapies and with one another is still unclear. Ultimately patients relapse from all available options.

In many cases, the same agents used as induction therapy may be reinstituted for relapsed disease if the disease recurred more than 6 to 12 months after the last therapy ended. However, if the relapse is of shorter duration, the patient is refractory to initial therapy, or the disease is associated with severe symptoms like renal failure or hypercalcemia, a regimen with different mechanism of action (class switch) is often selected. Patients for whom stem cells were cryopreserved early in the disease course, and who are transplant candidates, may benefit from autologous stem-cell transplantation (ASCT) as salvage therapy (Cavo, M., et al. Blood (2011) Vol 117, pages 6063-6073).

Melflufen, or a salt thereof, for use according to the dosage regime of the present invention is applicable to any of the aforementioned categories and classes of multiple myeloma. It is very effective in the treatment of refractory, relapsed and refractory-relapsed multiple myeloma. For example the dosage regime of the present invention comprising administering melflufen is useful for patients refractory (e.g. refractory or refractory-relapsed) to a protease inhibitor (Pis), immunomodulatory drug (IMiDs) or alkylator. It is especially useful in patients that are refractory (e.g. refractory or refractory-relapsed) to an alkylator, for example one or more of low dose melphalan, high dose melphalan and cyclophosphamide. It is also useful for patients refractory to one or more (for example 1, 2, 3, 4 or 5 or more) drug from two of more classes of drugs selected from protease inhibitors (Pis), immunomodulatory drugs (IMiDs) or alkylators.

Melflufen for use according to the dosage regime of the present invention is also especially useful in patients that are refractory (e.g. refractory or refractory-relapsed) to at least one immunomodulatory drug (IMiDs), and more especially in patients that are refractory (e.g. refractory or refractory-relapsed) to at least the immunomodulatory drug lenalidomide, and more especially to at least lenalidomide and 2, 3 or 4 other drugs including at least one protease inhibitor (PI) and immunomodulatory drug (IMiD).

Melflufen for use according to the dosage regime of the present invention is also especially useful in patients that are refractory (e.g. refractory or refractory-relapsed) to at least pomalidomide and/or daratumumab.

A combination of melflufen, or a salt thereof, and dexamethasone for use according to the dosage regime of the present invention is very useful in the treatment of refractory, relapsed and refractory-relapsed multiple myeloma, and more especially in the treatment of refractory-relapsed multiple myeloma. For example the dosage regime of the present invention comprising administering melflufen and dexamethasone, is useful for patients refractory (e.g. refractory or refractory-relapsed) to a protease inhibitor (Pis), immunomodulatory drug (IMiDs) or alkylator. It is especially useful in patients that are refractory (e.g. refractory or refractory-relapsed) to an alkylator, for example one or more of low dose melphalan, high dose melphalan and cyclophosphamide. It is also useful for patients refractory to one or more (for example 1, 2, 3, 4 or 5 or more) drug from two of more classes of drugs selected from protease inhibitors (Pis), immunomodulatory drugs (IMiDs) or alkylators. The dosage regime of the present invention comprising administering melflufen and dexamethasone, is also especially useful in patients that are refractory (e.g. refractory or refractory-relapsed) to at least one immunomodulatory drug (IMiD), and more especially in patients that are refractory (e.g. refractory or refractory-relapsed) to at least the immunomodulatory drug lenalidomide; and more especially to at least lenalidomide and 2, 3 or 4 other drugs including at least one protease inhibitors (Pis) and immunomodulatory drugs (IMiDs). The dosage regime of the present invention comprising administering melflufen and dexamethasone, is also especially useful in patients that are refractory (e.g. refractory or refractory-relapsed) to at least pomalidomide and/or daratumumab.

### Examples

### 1. General:

### 1.1 Eligibility for Inclusion in the Example 1 and 2 Clinical Trials

| | |
|---|---|
| Ages Eligible for Study: | 18 Years and older |
| Genders Eligible for Study: | Both |
| Accepts Healthy Volunteers: | No |

### 1.2 Criteria for Inclusion in the Example 1 and 2 Clinical Trials

### 1.2(a) Inclusion Criteria:

1. Male or female, age 18 years or older
2. Patient has a diagnosis of multiple myeloma with documented relapsed and/or relapsed-refractory disease
3. Patient has measurable disease defined as any of the following:
   a. Serum monoclonal protein ≥ 0.5 g/dL by protein electrophoresis
   b. ≥200 mg of monoclonal protein in the urine on 24-hour electrophoresis
   c. Serum immunoglobulin free light chain ≥10 mg/dL AND abnormal serum immunoglobulin kappa to lambda free light chain ratio
   d. If no monoclonal protein is detected, then ≥ 30% monoclonal bone marrow plasma cells
4. Patient has had at least 2 or more prior lines of therapy including lenalidomide and bortezomib and has demonstrated disease progression on or within 60 days of completion of the last therapy
5. Life expectancy of ≥6 month
6. Patient has an ECOG performance status ≤ 2 (Patients with lower performance status based solely on bone pain secondary to multiple myeloma will be eligible)
7. Females of childbearing potential must have a negative serum or urine pregnancy test prior to patient registration
8. Female patients of child bearing potential and non-vasectomized male patients agree to practice appropriate methods of birth control
9. Ability to understand the purpose and risks of the study and provide signed and dated informed consent and authorization to use protected health information
10. The patient has, or accepts to have, an acceptable infusion device for infusion of melflufen
11. 12 lead ECG with QtcF interval ≤ 470 msec
12. The following laboratory results must be met within 21 days of patient registration:
   ∘ Absolute neutrophil count ≥ 1,000 cells/dL (1.0 x 109/L)
   ∘ Platelet count ≥ 75,000 cells/dL (75 x 109/L)
   ∘ Hemoglobin ≥ 8.0 g/dL
   ∘ Total Bilirubin ≤ 1.5 x upper limit of normal
   ∘ Renal function: Estimated creatinine clearance ≥ 45 ml/min or serum creatinine ≤ 2.5 mg/dL
   ∘ AST (SGOT) and ALT (SGPT) ≤ 3.0 × ULN

### 1.2(b) Exclusion Criteria:

1. Patient has evidence of mucosal or internal bleeding and/or is platelet transfusion refractory
2. Any medical conditions that, in the Investigator's opinion, would impose excessive risk to the patient or would adversely affect his/her participation in this study
3. Known active infection requiring parenteral or oral anti-infective treatment
4. Other malignancy within the past 3 years with the exception of adequately treated basal cell carcinoma, squamous cell skin cancer, carcinoma in-situ of the cervix
5. Other ongoing anti-myeloma therapy. Patients may be receiving concomitant therapy with bisphosphonates and low dose corticosteroids for symptom management and comorbid conditions. Doses of corticosteroid should be stable for at least 7 days prior to patient registration.
6. Pregnant or breast-feeding females
7. Serious psychiatric illness, active alcoholism, or drug addiction that may hinder or confuse follow-up evaluation
8. Known HIV or hepatitis B or C viral infection
9. Patient has concurrent symptomatic amyloidosis or plasma cell leukaemia
10. POEMS syndrome
11. Previous cytotoxic therapies, including cytotoxic investigational agents, for multiple myeloma within 3 weeks (6 weeks for nitrosoureas) prior to start of study treatment. Biologic, novel therapy (including investigational agents in this class) or corticosteroids within 2 weeks prior to patient registration. Patient has side effects of the previous therapy ≥ grade 1 or previous baseline.
12. Prior peripheral stem cell transplant within 12 weeks of patient registration
13. Radiotherapy within 21 days prior to Cycle 1 Day 1. However, if the radiation portal covered ≤ 5% of the bone marrow reserve, the patient may be enrolled irrespective of the end date of radiotherapy
14. Known intolerance to steroid therapy

### 1.3 Treatment regimen for Example 1 and 2 Clinical Trials

Example 1: 4 dose levels (15, 25, 40 and 55 mg) of intravenous melflufen hydrochloride (excluding the mass of the salt component) in 290 to 370 ml glucose solution administered over 30 minutes on Day 1 of a 21-day cycle (in combination with dexamethasone treatment on Days 1, 8 and 15), for at least 1 cycle and up to 12 cycles, were evaluated.

Example 2: An open label single arm extension on the dose of 40 mg over 30 minutes of Example 1. Intravenous melflufen was administered over 30 minutes on Day 1 of a 21-day cycle or 28-day cycle (in combination with 40 mg dexamethasone (oral or intravenous) treatment on Days 1, 8 and 15) for at least 2 cycles, and up to the stated number of cycles. The cycle length was extended during the Example 2 Clinical Trial from 21 to 28 days per a protocol amendment to allow for a better recovery of neutrophils and thrombocytes before a new cycle is initiated.

Dose reductions from 40 mg to 25 mg melflufen for patients in the trial was possible in connection with adverse effects of thrombocytopenia/neutropenia.

Example 2a results are from a data cut-off of time point (a). Example 2b results are from a data cut-off of time point (bi) for the efficacy data, which was approximately 12 months after time point (a); and from a data cut-off of time point (bii) for the safety data, which was approximately 10 months after time point (a).

### 1.4 Drug substance

Melflufen hydrochloride is obtainable as described in WO 01/96367. Melflufen hydrochloride powder for solution for infusion used in Examples 1 and 2 was provided as a white, freeze dried (lyophilized) solid in 15 mg or 25 mg vial strengths (the mass of the melflufen hydrochloride excludes the mass of the salt component).

Before administration, the melflufen hydrochloride powder for solution for infusion in each vial is dissolved in 40 ml of 5 % glucose solution, and is mixed by shaking the vial. The solution in the vial is then injected into an infusion bag of 250 ml of 5% glucose solution. To arrive at the desired dosage in one infusion bag, the solution in more than one vial (e.g. two vials or three vials) may be injected into the infusion bag. For example, to arrive at a 40 mg dosage, the solution from a 15 mg vial and the solution from a 25 mg vial may be added to one infusion bag of 250 ml of 5% glucose solution (resulting in a total volume of 330 ml in the infusion bag).

Dexamethasone in pharmaceutical grade is obtainable from many suppliers.

### 2. Example 1

### 2.1 Clinical Pharmacokinetic Study

The pharmacokinetic (PK) behaviour of melflufen in man (provided as a melflufen hydrochloride salt) and the metabolites melphalan and des-ethyl-melflufen were studied. A preliminary analysis of PK data in six patients was performed. The participants had a diagnosis of multiple myeloma and they fulfilled the inclusion criteria set out above. PK parameters by patient are shown in Table 1 and representative concentration-time profiles for the compounds in one patient dosed at 25 mg, one patient dosed at 40 mg, and one patient dosed at 55 mg of melflufen are shown are shown in Figure 1(a), 1(b) and 1(c) respectively.

**Table 1: PK Parameters for Melflufen and its Metabolites Melphalan and Des-ethyl-Melflufen by Subject in Example 1**

| Dose melflufen HCl (mg) | | 15 | 25 | 25 | 40 | 40 | 55 |
|---|---|---|---|---|---|---|---|
| Melphalan | T_{1/2} (h) | 1.31 | 1.28 | 1.38 | 1.21 | 1.52 | 1.30 |
| | tₘₐₓ (min) | 31 | 37 | 38 | 35 | 40 | 41 |
| | Cₘₐₓ (ng/mL) | 173 | 562 | 398 | 508 | 480 | 1050 |
| | AUC_{inf} (ng/m L*h) | 387 | 1233 | 757 | 835 | 875 | 2133 |
| | AUC₀₋₄ (ng/m L*h) | 332 | 1073 | 650 | 745 | 725 | 1848 |
| Melflufen | T_{1/2} (min) | 13 | 4.6 | 3.9 | 1.1 | 1.8 | 2.7 |
| | tₘₐₓ (min) | 15 | 12 | 15 | 15 | 25 | 25 |
| | Cₘₐₓ (ng/mL) | 39 | 102 | 158 | 222 | 160 | 245 |
| | AUC_{inf} (ng/m L*h) | 16.3 | 40.8 | 58.9 | 89.3 | 50.2 | 102 |
| | AUC₀₋₄ (ng/m L*h) | 13.5 | 40.8 | 54.9 | 89.3 | 50.2 | 102 |
| Des-ethyl-melflufen | T_{1/2} (min) | 27 | 17 | 13 | 6.0 | 3.6 | 13 |
| | tₘₐₓ (min) | 29 | 25 | 29 | 25 | 25 | 29 |
| | Cₘₐₓ (ng/mL) | 6.5 | 12.3 | 13.0 | 12.3 | 10.3 | 16.6 |
| | AUC_{inf} (ng/m L*h) | 4.46 | 9.08 | 9.44 | 5.50 | 3.73 | 9.77 |
| | AUC₀₋₄ (ng/m L*h) | 1.51 | 9.07 | 4.05 | 5.50 | 3.73 | 9.77 |

As can be seen from the above data and Figure 1, during administration of melflufen as an intravenous infusion over 30 minutes, melflufen concentrations reached an early plateau or started to decrease during the latter part of the infusion. After end of infusion, melflufen concentrations decreased with a half-life in the order of 3 to 5 minutes and was no longer measurable within 15 minutes.

Melphalan was rapidly formed and reached plasma concentrations higher than those of melflufen by the first measurement point (15 minutes of start of melflufen infusion). After the end of the melflufen infusion, melphalan plasma concentrations continued to increase for up to 10 minutes and, thereafter, decreased at a rate similar to what was typically observed after an infusion of melphalan ("Alkeran prescribing information." US Food and Drug Administration. FDA (2012)).

This delay in peak plasma concentration of melphalan is compatible with an extensive formation of melphalan from melflufen in peripheral tissues with subsequent distribution of melphalan back to blood. Estimated melphalan clearance after administration of melflufen was of the same magnitude as in published studies with direct administration of equimolar doses of melphalan, indicating a close to complete conversion of melflufen to melphalan (Nath, C. E., et al. Br J Clin Pharmacol (2010) Vol 69, pages 484-497). The metabolite des-ethyl-melflufen reached only very low concentrations in plasma and was eliminated with a half-life of approximately 15 minutes or less.

Similar PK results and conclusions were also seen in a clinical trial administering from 15 mg to 130 mg melflufen hydrochloride (excluding the mass of the salt component) over 30 minutes in patients with different cancer types.

### 2.2 Discussion of Pharmacokinetic Data

The results from Example 1 described above demonstrate that the PK of melflufen is characterized by low plasma concentrations and a very rapid disappearance from plasma after end of the intravenous infusion over 30 minutes, with a half-life of 3 to 5 minutes. The PK of melphalan after administration of melflufen is characterized by a rapid formation, where plasma concentrations exceed those of melflufen within 15 minutes after start of melflufen infusion, but where peak plasma concentrations were lower than after equimolar infusions of melphalan at a similar rate (Mougenot, P., et al, Cancer Chemother Pharmacol (2004)) Vol 53, pages 503-512., Nath, C. E., et al. Br J Clin Pharmacol (2010) Vol 69, pages 484-497). Peak plasma concentrations of melphalan appeared with a delay by up to 10 minutes after the end of the melflufen infusion. After the melflufen infusion, AUC and elimination half-life for melphalan were in accordance with those observed after equimolar doses of melphalan (Mougenot, P., et al, Cancer Chemother Pharmacol (2004)) Vol 53, pages 503-512., Nath, C. E., et al. Br J Clin Pharmacol (2010) Vol 69, pages 484-497).

Overall, the observations suggested a mechanism where melflufen is rapidly and widely distributed to tissues or blood components outside of the plasma compartment, where melphalan is formed and, thereafter, distributed back to plasma. There are no signs of redistribution of melflufen back to plasma. The PK of melphalan, including the influence of patient factors, has been previously extensively characterized during melphalan therapy.

As discussed above, melflufen is very rapidly distributed out of the plasma compartment and thereafter metabolized to melphalan in cells and tissues. The conversion of melflufen into melphalan occurs primarily intracellularly and is catalysed by peptidases and esterases. Renal elimination of melflufen or hepatic metabolism is unlikely to contribute to melflufen elimination since the rapid disappearance from plasma and local metabolism prohibits melflufen from reaching these organs in any meaningful amounts.

The relationship between renal function and melphalan pharmacokinetics has been evaluated in two smaller studies that included 11 to 15 patients (Adair, C. G., et al, Cancer Chemother Pharmacol (1986) Vol 17, pages 185 - 188; Osterborg, A., et al, Eur J Cancer Clin Oncol (1989) Vol 25, pages 899-903), and in a population PK clinical trial with 100 patients (Nath, C. E., et al. Br J Clin Pharmacol (2010) Vol 69, pages 484-497).

In those studies, very few patients had a glomerular filtration rate (GFR) < 30 mL/min. Results were consistent across studies and demonstrated a slightly less than two-fold increase in melphalan AUC and elimination half-life when GFR decreased from 120 mL/min to 30 mL/min. The melphalan prescribing information states that dosage reduction of up to 50% should be considered in patients with renal insufficiency. As melflufen appears to be completely metabolized to melphalan, the AUC and elimination half-life of melphalan are likely to increase with impaired renal function to a similar extent during treatment with melflufen.

However, the specific advantage with melflufen when administered in accordance with the invention is the rapid distribution to tissues with local metabolism to melphalan resulting in high intracellular melphalan concentrations. The melflufen dosage does not need to be reduced in patients with impaired renal function. It is expected that the distribution of melflufen and local metabolism to melphalan are not likely to be affected by the renal function. A longer elimination half-life for melphalan will not lead to accumulation as melflufen is administered as a single 30 minute infusion with an interval of at least 7 days.

### 2.3. Safety of Various Dosages of Melflufen

Treatment emergent adverse events (TEAEs) of any grade were recorded in 29 patients that took part in Example 1 (4 patients at 15 mg dosage of melflufen over 30 minutes; 7 patients at 25 mg dosage of melflufen over 30 minutes; 12 patients at 40 mg dosage of melflufen over 30 minutes; and 6 patients at 55 mg dosage of melflufen over 30 minutes).

A total of 157 TEAEs were reported in 26 of the 29 patients in the study. The most frequent TEAEs occurring at least once in a specific patient, all grades, regardless of relationship to study drug included thrombocytopenia (69% of the total number of TEAEs, reported in 20 patients), anemia (59%, reported in 17 patients), neutropenia (41%, reported in 12 patients) and nausea (38%, reported in 11 patients). Grade 3 or 4 TEAEs, regardless of relationship to study drug, have been reported in 76% of patients (in 22 out of 29 patients). All events have been reported as common in connection with treatment with alkylators including melphalan.

Total number of treatment-related Grade 3 and 4 TEAEs recorded in at least 2 patients at the various dose levels used in Example 1 are shown in Table 1, as well as Grade 3 and 4 thrombocytopenia, neutropenia and febrile neutropenia events. The most common related Grade 3 or 4 TEAEs were reversible thrombocytopenia and neutropenia, which occurred at least once in 41% and 38% of the patients respectively. More of these bone marrow related events occurred in the 55 mg group compared with the other groups.

**Table 2: All treatment-related Grade 3 and 4 TEAEs, Grade 3 and 4 thrombocytopenia, neutropenia and febrile neutropenia events reported at each dose level in Example 1**

| | 15 mg (n = 4) n (total number of events) | 25 mg (n = 7) n (total number of events) | 40 mg (n = 12) n (total number of events) | 55 mg (n = 6) n (total number of events) |
|---|---|---|---|---|
| Any | 2 (4) | 5 (13) | 7 (23) | 6 (58) |
| Thrombocytopenia | 1(1) | 2 (3) | 4 (9) | 5 (28) |
| Neutropenia | 0 (0) | 2 (6) | 4 (8) | 5 (17) |
| Febrile neutropenia | 1 (1) | 0 (0) | 1 (2) | 1 (1) |

### 3. Example 2a

Example 2a is the data/results of Clinical Trial Example 2 at time point (a) during the clinical trial.

### 3.1 Efficacy Data from Study in Patients with RRMM

By the data cut-off point for Example 2a, 38 patients with relapsing MM had been dosed with 40 mg of melflufen hydrochloride (the 40 mg dosage excludes the mass of the salt component) administered over 30 minutes every 3 weeks (21 days) in combination with weekly dexamethasone (day 1, 8 and 15). 162 doses of melflufen were administered in total. The median number of cycles initiated was 3 (1-13) and the median duration of treatment was 13 weeks (2-51). The mean dose intensity was 96% (77-100). By the data cut-off point, ten patients were still in treatment, 2 had completed treatment and 26 patients discontinued from treatment (15 due to AEs, 8 due to PD, 2 deaths and 1 for other reasons). Twenty-seven patients were still in the study (10 patients in treatment and 17 in follow-up), while 11 patients were off study (8 patients due to death, 1 due to PD, 1 withdrew consent and 1 lost in follow-up).

Twenty-seven patients were evaluable for efficacy (per the protocol, these patients had received at least two cycles of melflufen and had adequate follow-up assessments). 11 patients were not evaluable for response due to rapid early progression (7), early termination due to adverse events (3) or too early to assess (1).

A summary of the baseline characteristics of the 38 patients in the study, including the efficacy evaluable patients, are shown in Table 3 below. 66% had International Staging System (ISS) stage II-III Multiple Myeloma and 26% had high risk cytogenetic risk factors by cytogenetic risk factors by (FISH), 47% standard risk and 27% were not done/unknown. The patients had a median 5 years (1-15) since diagnosis and a median of 4 (2-9) prior lines of therapy. 62% were double-refractory to an IMiD and PI and 57% were refractory to an alkylator.

**Table 3: Baseline Characteristics of Patients in Example 2a**

| Characteristics | | Total | Efficacy evaluable (n = 27) | |
|---|---|---|---|---|
| | | N = 38 | Responders (n = 11) | Non-responders (n = 16) |
| Median age, years (range) | | 65 (47-76) | 68 (48-74) | 63 (47 - 73) |
| ≥ 75 years, n (%) | | 2 (5) | 0 | 0 |
| Years since diagnosis, median (range) | | 5** (1-15) | 7 (7-15)* | 5 (1-14) |
| Number of previous lines of therapy, median (range) | | 4 (2-9) | 4 (3-7) | 3 (2-6) |
| ISS stage, n (%) | | | | |
| | I | 11 (29) | 5 (45) | 6 (38) |
| | II or III | 25 (66) | 5 (45) | |
| Unknown | | 2(5) | 1(9) | 0 |
| | ECOG performance status, n (%) | | | |
| | 0 | 16(42) | 6 (55) | 8 (50) |
| | 1 | 20 (53) | 5 (45) | 6 (38) |
| | Not done | 2 (5) | 0 | 2 (13) |

| Cytogenetic risk factor by FISH, n (%) | | | | |
|---|---|---|---|---|
| | High [del(17)p13, t(4;14)(p16;q32) or t(14; 16)(q32;q23)] | 10 (26) | 2 (18) | 5 (31) |
| | Standard | 18 (47) | 5 (45) | 8 (50) |
| | Not done | 9 (24) | 3(37) | 3(19) |
| | Unknown | 1 (3) | 1 (9) | 0 |
| Double-refractory (IMiD and PI)***, n (%) | | 23* (62) | 5 (45) | 11 (69) |
| Refractory to melphalan, cyclophosphamide or bendamustine, n (%) | | 21* (57) | 8 (73) | 5 (55) |
| *Missing information on one patient | | | | |
| "Missing information on two patients | | | | |
| ***16 patients (59%) were double-refractory in the efficacy evaluable population (N=27) | | | | |

Of the 27 patients evaluable for efficacy, 15 showed a best response of Minimal Response (MR) or better: 2 patients achieved very good partial response (VGPR) and 9 achieved partial response (PR) for an ORR of 41%. Four additional patients achieved minimal response (MR) for a clinical benefit rate (CBR) of 56%. Table 4 summarises these results. Table 4 also shows the results in 35 patients treated with one or more cycle of melflufen.

**Table 4: Efficacy Data from Patients Treated with 40 mg Melflufen in Example 2a**

| | n | Very Good Positive Response (PR) | Positive Response (PR) | Minimal Response (MR) | Stable Disease (SD) | Progressive Disease (PD) | Overall Response Rate (ORR) | Clinical Benefit Rate (CBR) |
|---|---|---|---|---|---|---|---|---|
| Evaluable ≥ 2 cycles | 27 | 2 | 9 | 4 | 11 | 1 | 41% | 56% |
| Evaluable ≥ 1 cycle | 35 | 2 | 9 | 4 | 12 | 8 | 31% | 43% |

The overall ORR is evaluable patients is 41% and CBR is 56%.

Figure 2 shows the change in para-protein levels for the 27 patients' evaluable for efficacy. Paraproteins are produced in large amounts by abnormal myeloma cells, and thus are an indicator of the activity of multiple myeloma: paraprotein levels will fall with successful treatment. As can be seen from Figure 2, paraprotein levels decreased in 22 of the 27 patients, with reductions of over 50 % in 12 patients, and over 90 % in 4 patients.

Figure 3 shows a Kaplan-Meier Plot of progression free survival (PFS) for all patients in Example 2a treated with at least one dosage of 40 mg melflufen hydrochloride as an intravenous dosage over 30 minutes ("ALL") (n=38), and the efficacy evaluable patient ("PP") as described above (n = 27). The median progression free survival (PFS) was 9.4 months (95% CI: 3.7 to ∞) based on 13 events in 27 patients.

Figure 4 also shows the Kaplan-Meier Plot of progression free survival (PFS) for all patients in Example 2a treated with at least one dosage of 40 mg melflufen hydrochloride (n=38), and additionally shows the Kaplan-Meier Plot of PFS for patients in the phase II clinical trial of pomalidomide (San Miguel, J., et al., Lancet Oncol, (2013), Vol 14, pages 1055-1066). The median PFS for pomalidomide is 4.0 months. As can be seen, the long term PFS for melflufen is much higher than pomalidomide. The hazard ratio for melflufen compared to pomalidomide is 0.68 (0.44 - 1.05), i.e. there is a 32% reduction in risk of death over 16 months when using melflufen compared to pomalidomide in a RRMM patient population.

Figure 5 shows a Kaplan-Meier Plot of duration of response (DOR) in the 11 patients who responded to treatment (PR or better). The median duration of response (DOR) was 9.6 months (95% CI: 7.1 to ∞) based on 4 events in 11 patients.

It is important that the clinical response data in MM clinical trials are interpreted in the context of information about the patients' previous treatment exposure and their refractoriness to previous treatments. Table 5 summarizes refractory status of the efficacy evaluable patients (based on the IMWG (Palumbo, A., et al, J Clin Oncol (2014) Vol 32, pages 587-600) definition: relapsed on or within 60 days of last dose of treatment) in the 27 patients evaluable for efficacy. Prior to entry into the clinical trial, 26 of these were refractory to at least one class of PIs, IMiDs and alkylators. 16 patients (59%) were double-refractory (PI+IMiD) and 14 patients (52%) were alkylator-refractory.

Of the 14 patients who were previously shown to be refractory to alkylator treatment were included in the clinical trial. 9 of these patients were shown refractory to cyclophosphamide, 4 to low dose melphalan, and 3 to high dose melphalan.

Table 5 also summarizes ORR and clinical benefit rate (CBR) per refractory status patient subgroup.

**Table 5: Refractory Status at Baseline; Overall Response Rate (ORR) and Clinical Benefit Rate (CBR) based on Refractory Status of the 27 Patients Evaluable for Efficacy in Example 2a**

| Refractory Status | | n (%) | ORR (≥ PR) Total 11 of 27 (41%) | CRR (≥ MR) Total 15 of 27 (56%) |
|---|---|---|---|---|
| None | | 1 (4) | 1 (100) | 1 (100) |
| Pl | | 17 (63) | 6 (35) | 8 (47) |
| IMiD | | 23 (85) | 8 (35) | 12 (52) |
| Alkylator | | 14 (52) | 8 (57) | 9 (64) |
| | Low dose melphalan | 4 | 2 | 3 |
| | High dose melphalan | 3 | 2 | 3 |
| | Cyclophospham ide | 9 | 6 | 6 |
| PI + IMiD | | 16 (59) | 5 (31) | 7 (44) |
| PI + IMiD + Alkylator | | 9 (33) | 3 (33) | 4 (44) |
| Triple refractory (2 PI/ImiD + 1 ImiD/PI) | | 10 (37) | 4 (4) | 6 (60) |
| Pomalidomide refractory | | 10 (37) | 3 (30) | 5 (50) |

Similar ORR to the overall ORR (41%) were seen in PI-refractory (35%), IMiD-refractory (35%), alkylator-refractory (57%), double-refractory (31%) and triple-refractory (40%) patients. Thus, good results were seen across patient populations regardless of refractory status.

Of the 11 patients who had a confirmed PR after melflufen treatment, 5 patients were documented to be double-refractory (PI+IMiD). The response rate in this double-refractory population was, thus, similar to the response rate in the whole clinical trial population, where 11 out of 27 patients had a confirmed PR.

Also of note is that 8 of the 11 responding patients were alkylator-refractory and that 8 out of the 14 alkylator-refractory patients in the clinical trial responded with a PR. These data suggest that melflufen has significant efficacy in alkylator-refractory disease.

Thus, melflufen has promising activity in heavily pre-treated RRMM patients where conventional therapies have failed, and especially in alkylator-refractory patients. ORR and CBR based on number of prior refractory agents in efficacy evaluable patients (n = 27) is shown in Table 6. Responders and non-responders do not show major differences in number of previous therapies.

These data show that there is a substantial and durable treatment effect from 40 mg melflufen (as melflufen hydrochloride) administered over 30 minutes in this heavily pre-treated and highly refractory MM population.

**Table 6: ORR and CBR based on number of prior refractory agents in efficacy evaluable patients in Example 2a (n = 27)**

| Number of refractory agents* | n (%) | ORR (≥ PR) Total 11 of 27 (41%) | CBR (≥ MR) Total 15 of 27 (56%) |
|---|---|---|---|
| 0 | 1 (4) | 1 (100) | 1 (100) |
| 1 | 26 (96) | 10 (38) | 14 (54) |
| 2 | 21 (78) | 9 (43) | 12 (57) |
| 3 | 16 (59) | 7 (44) | 10 (63) |
| 4 | 7 (26) | 3 (43) | 5 (71) |
| * not counting steroids | | | |

### 3.2 Safety Data in Study in Patients with RRMM

As mentioned above, by the data cut-off for Example 2a 38 patients had been dosed with 162 doses of melflufen hydrochloride 40 mg over 30 minutes. Median number of cycles was 3 (1-13) and median duration of treatment was 13 weeks (2-51 weeks). The dose intensity was 96% (77-100).

Ten patients were still in treatment, 2 had completed treatment (≥ 8 cycles of therapy) and 26 patients discontinued from treatment (15 due to AEs, 8 due to PD, 2 deaths and 1 for Cachexia in progressive disease).Twenty-seven patients were still in the study (10 patients on treatment and 17 in follow-up), while 11 patients were off study (8 patients due to death, 1 due to PD, 1 withdrew consent and 1 lost in follow-up).

All 38 patients experienced drug related treatment emergent adverse events (TEAEs) of any grade. Thirty-four patients (90%) experienced grade 3 or 4 TEAEs, and 33 (87%) patients experienced treatment-related grade 3 or 4 TEAEs.

The incidences of ≥ Grade 3 and Grade 4 TEAEs reported in >5% of patients (n=38) receiving melflufen hydrochloride 40 mg throughout all treatment cycles are shown in Table 7.

Also provided in Table 7 are a summary of the Grade ≥3 and Grade 4 TEAEs in 6 patients dosed with a higher melflufen hydrochloride dose (55 mg) in Example 1. The TEAEs are assessed as related to the study treatment. In the 55 mg melflufen hydrochloride dose group, all patients developed Grade 3 or Grade 4 neutropenia and 5 patients out of 6 patients developed Grade 3 or Grade 4 thrombocytopenia. As shown in Table 7, the incidences of thrombocytopenia and neutropenia were much lower for the 40 mg melflufen hydrochloride dose.

The occurrences of other ≥Grade 3 and Grade 4 TEAEs (i.e. excluding thrombocytopenia and neutropenia) were low for the 40 mg dosage of melflufen hydrochloride. Hematologic toxicity was common, but non-hematologic TEAEs were infrequent.

It was found that the safety profile for melflufen is similar to that for other alkylators, with neutropenia and thrombocytopenia as the most common AEs.

**Table 7: Treatment Related ≥ Grade 3 TEAEs Reported in >5% of Patients in Example 2a (N=38)**

| System Organ Class *(Preferred Term)* | | 40 mg melflufen hydrochloride | | 55 mg melflufen hydrochloride |
|---|---|---|---|---|
| | | Treatment related ≥grade 3 n (%) | Treatment related grade 4 n (%) | Treatment related ≥grade 3 n (%) |
| Any treatment-related grade 3 and/or grade 4 | | 33 (87) | 19 (50) | 6 (100) |
| Blood and lymphatic system disorders | | 29 (76) | 18 (47) | 6 (100) |
| | *Thrombocytopenia* | 28 (68) | 13 (34) | 5 (83) |
| | *Neutropenia* | 20 (53) | 10 (26) | 6 (100) |
| | *Anemia* | 15 (40) | 0 | 2 (33) |
| | *Leukopenia* | 13 (34) | 5 (13) | 0 |
| | *Febrile neutropenia* | 2 (5) | 0 | 0 |
| | *Pancytopenia* | 0 | 0 | 1 (17) |
| General disorders and administration site conditions | | 7 (18) | 0 | 1 (17) |
| | *Asthenia* | 2 (5) | 0 | 0 |
| | *Fatigue* | 2(5) | 0 | 1 (17) |
| | *Pyrexia* | 2 (5) | 0 | 0 |
| Infections and infestations | | 3 (8) | 0 | 3 (50) |
| | *Pneumonia* | 3 (8) | 0 | 2 (33) |
| | *Sepsis* | | | 1 (17) |
| | *Urinary tract infection* | | | 1 (17) |
| Investigations | | 4 (10) | 0 | 0 |
| | *Neutrophil count decreased* | 4 (10) | 0 | 0 |
| Metabolism and nutrition disorders | | 3 (8) | 1 (3) | 0 |
| | *Hyperglycemia* | 3 (8) | 1 (3) | 0 |
| Skin and subcutaneous disorders | | 0 | 0 | 1 (17) |
| | *Cutaneous rash* | 0 | 0 | 1 (17) |

Of the 38 patients dosed with 40 mg melflufen in Example 2a, thirteen patients (34%) experienced Serious TEAEs and 8 patients (21%) experienced treatment-related Serious TEAEs (Table 8). Seven patients (18%) had TEAEs leading to dose reduction of melflufen. Three patients (8%) had TEAEs leading to death.

**Table 8 Serious TEAEs Related to Melflufen 40 mg in Example 2a**

| Adverse Event Term | Number of patients (%) |
|---|---|
| *Pneumonia* | 3 (8) |
| Febrile Neutropenia | 2 (5) |
| Pyrexia | 2 (5) |
| Diarrhoea | 1 (3) |
| Escherichia coli sepsis | 1 (3) |
| Neutropenia | 1 (3) |

### 4. Example 2b

Example 2b is the data/results of Clinical Trial Example 2 at:
time point (bi) for the efficacy data, which was approximately 12 months after time point (a); and
time point (bii) for the safety data, which was approximately 10 months after time point (a).

The data from example 2b was taken at time point (bi) for the efficacy data during the clinical trial, which was approximately 12 months after time point (a); and at time point (bii) for the safety data during the clinical trial, which was approximately 10 months after time point (a);

### 4.1 Efficacy Data from Study in Patients with RRMM at time point (bi)

By the data cut-off point (bi), 40 patients with relapsing MM had been dosed with 40 mg of melflufen hydrochloride (the 40 mg dosage excludes the mass of the salt component) administered over 30 minutes every 3 weeks (21 days) in combination with weekly dexamethasone (day 1, 8 and 15) or every 4 weeks (28 days) in combination with weekly dexamethasone (day 1, 8, 15 and 22).

30 patients were evaluable for efficacy (per the protocol, these patients had received at least two cycles of melflufen and had adequate follow-up assessments). 10 patients were not evaluable for efficacy evaluation due to rapid early progression (8), or early termination due to adverse events (2).

Of the 30 patients evaluable for efficacy, 15 showed a best response of Minimal Response (MR) or better: 2 patients achieved very good partial response (VGPR) and 9 achieved partial response (PR) for an ORR of 41%. Four additional patients achieved minimal response (MR) for a clinical benefit rate (CBR) of 56%. Table 9 summarises these results. Table 9 also shows the results in the 40 patients treated with one or more cycle of melflufen.

**Table 9: Efficacy Data from Patients Treated with 40 mg Melflufen in Example 2b**

| | n | Very Good Positive Response (PR) | Positive Response (PR) | Minimal Response (MR) | Stable Disease (SD) | Progressive Disease (PD) | Overall Response Rate (ORR) | Clinical Benefit Rate (CBR) |
|---|---|---|---|---|---|---|---|---|
| Evaluable ≥ 2 cycles | 30 | 3 | 9 | 7 | 10 | 1 | 40% | 63% |
| Evaluable ≥ 1 cycle | 40 | 3 | 9 | 8 | 11 | 9 | 30% | 50% |

The overall ORR for evaluable patients is 40% and CBR is 63%.

By the data cut-off, the median duration of response (DOR) was 7.7 months (95% confidence interveral, 4.6 months to ∞) based on 11 events in 12 patients, 1 patient was still alive, had not progressed and was therefore censored at the latest time of tumor assessment. This analysis has been performed in all responding patients (≥PR).

Figure 6 shows a Kaplan-Meier Plot of progression free survival (PFS) for all patients in Example 2b treated with at least one dosage of 40 mg melflufen hydrochloride as an intravenous dosage over 30 minutes ("ITT") (n=40), and the efficacy evaluable patient ("PP") as described above (n=30). The median progression free survival (PFS) in the PP-population was 7.9 months (95% CI: 4.1 to 12 months) based on 25 events in 30 patients. 5 patients were still alive, had not progressed and were therefore censored at the latest time of tumor assessment. The median progression free survival (PFS) in the ITT-population was 4.3 months (95% CI: 3.7 to 9.5 months) based on 34 events in 40 patients with available data. 6 patients were still alive, had not progressed and were therefore censored at the latest time of tumour assessment. These data suggest that the responses could be of considerable duration and that also patients with MR and SD may have a benefit of considerable duration until progression.

As also noted in Section 3.2, it is important that the clinical response data in MM clinical trials are interpreted in the context of information about the patients' previous treatment exposure and their refractoriness to previous treatments. Table 10a summarizes refractory status of the efficacy evaluable patients in Example 2b (data cut-off (bi)) point based on the IMWG (Palumbo, A., et al, J Clin Oncol (2014) Vol 32, pages 587-600) definition: relapsed on or within 60 days of last dose of treatment) in the 29 patients evaluable for efficacy and who had data for assessment for refractoriness (missing refractoriness data for 1 of the 30 patients). Prior to entry into the clinical trial, 28 of these were refractory to at least one class of PIs, IMiDs and alkylators. 17 patients (59%) were double-refractory (PI+IMiD) and 15 patients (52%) were alkylator-refractory.

15 patients who were previously shown to be refractory to alkylator treatment were included in the clinical trial. 10 of these patients were shown refractory to cyclophosphamide, 5 to low dose melphalan, and 3 to high dose melphalan.

Table 10a also summarizes ORR and clinical benefit rate (CBR) per refractory status patient subgroup.

**Table 10a: Refractory Status at Baseline; Overall Response Rate (ORR) and Clinical Benefit Rate (CBR) based on Refractory Status of the 29 Patients Evaluable for Efficacy in Example 2b**

| Refractory Status^{b} | | Efficacy evaluable patients (N=29), n (%) | ORR (≥ PR) Total 12 of 30 (40%) | CRR (≥ MR) Total 19 of 30 (63%) |
|---|---|---|---|---|
| None | | 1 (3) | 1 (100) | 1 (100) |
| PI | | 19 (66) | 7 (37) | 12 (63) |
| IMiD | | 24 (83) | 9 (38) | 15 (63) |
| Alkylator | | 15 (52) | 8 (53) | 11 (73) |
| | Low dose melphalan | 5 (17) | 2 (40) | 3 (60) |
| | High dose melphalan | 3 (10) | 2 (67) | 3 (100) |
| | Cyclophosphamide | 10 (34) | 6 (60) | 8 (80) |
| PI + IMiD | | 17 (59) | 6 (35) | 10 (59) |
| PI + IMiD + Alkylator | | 9 (31) | 3 (33) | 4 (44) |
| Triple refractory (2 PI/ImiD + 1 ImiD/PI) | | 10 (34) | 3 (30) | 7 (70) |
| Pomalidomide refractory | | 11 (38) | 4 (36) | 6 (55) |
| Antibody refractory | | 2 (5) | 0 | 1 (50)+ |
| ^{a} Based on available data for 29 of the 30 efficacy evaluable patients (1 patient with missing data) | | | | |
| ^{b} According to the IMWG definition (Rajkumar et al. 2011). | | | | |
| ^{c} One patient refractory to daratumumab and one patient to elotuzumab. | | | | |

Similar ORR to the overall ORR (40%) were seen in PI-refractory (37%), IMiD-refractory (38%), alkylator-refractory (53%), double-refractory (35%) and triple-refractory (30%) patients. Thus, good results were seen across patient populations regardless of refractory status.

Of the 12 patients who had a confirmed PR after melflufen treatment, 6 patients were documented to be double-refractory (PI+IMiD). The response rate in this double-refractory population was, thus, better than the response rate in the whole clinical trial population, where 12 out of 30 patients had a confirmed PR.

Also of note is that 8 of the 12 responding patients (i.e. those with a confirmed PR) were alkylator-refractory, so 8 out of the 15 alkylator-refractory patients in the clinical trial responded with a PR. Further, 5 of the 8 patients who were refractory to an alkylator as their last line of treatment showed a best response of PR or better following melflufen treatment (data not included in Table 10a).These data suggest that melflufen has significant efficacy in alkylator-refractory disease.Thus, melflufen has promising activity in heavily pre-treated RRMM patients and in highly refractory patients where conventional therapies have failed, and especially in alkylator-refractory patients.

Table 10b summarizes exposure to prior medication in the all treated 39 patients who had data for assessment of refractoriness. Prior to entry into the clinical trial, 36 of the 39 patients had been exposed to three classes of MM drugs (Pis, IMiDs and alkylators). Thirty-eight (38) of the 39 patients with available data were refractory to at least one class. Of the 39 patients with refractory data, 24 patients (62%) were double-refractory (PI+IMiD), 22 patients (56%) were alkylator-refractory and 15 patients (38%) were double- and alkylator-refractory. Thirty-two (32) patients (82%) were refractory to their last line of therapy. Refractory status was unknown for 1 patient at the time of data cut-off (time point (bi)).

**Table 10b: Refractory Status at Baseline; Overall Response Rate (ORR) and Clinical Benefit Rate (CBR) based on Refractory Status of the 39 Treated Patients in Example 2b**

| Refractory Status^{b} | | Efficacy evaluable patients (N=39), n (%) | ORR (≥ PR) Total 12 of 30 (30%) | CRR (≥ MR) Total 15 of 30 (50%) |
|---|---|---|---|---|
| None | | 1 (3) | 1 (100) | 1 (100) |
| PI | | 28 (72) | 7 (25) | 14 (50) |
| IMiD | | 32 (82) | 9 (28) | 15 (47) |
| Alkylator | | 22 (56) | 8 (36) | 12 (55) |
| | Low dose melphalan | 6 (15) | 2 (33) | 4 (67) |
| | High dose melphalan | 3 (8) | 2 (33) | 3 (100) |
| | Cyclophosphamide | 15 (38) | 6 (40) | 8 (53) |
| PI + IMiD | | 25 (62) | 6 (25) | 11 (46) |
| PI + IMiD + Alkylator | | 15 (38) | 3 (20) | 6 (40) |
| Triple refractory (2 PI/ImiD + 1 ImiD/PI) | | 16 (41) | 3 (25) | 7 (44) |
| Pomalidomide refractory | | 16 (41) | 4 (25) | 7 (44) |
| Antibody refractory | | 3 (7) | 0 | 1 (33) |
| ^{a} Based on available data for 29 of the 30 efficacy evaluable patients (1 patient with missing data) | | | | |
| ^{b} According to the IMWG definition (Rajkumar et al. 2011). | | | | |
| ^{c} One patient refractory to daratumumab and one patient to elotuzumab. | | | | |

In summary, the available efficacy results in the Example 2b are encouraging. The clinical data support that melflufen has preserved anti-tumor activity also in double-refractory and alkylator-refractory MM patients, which is a population that is similar to the patient population used in the pomalidomide pivotal trial. As discussed above, the current ORR data and PFS data indicate a significant treatment effect. In addition, based on the shape of the PFS curves in the pomalidomide pivotal trial and the ongoing melflufen trial, there is a signal that suggests that melflufen+dexamethasone may provide a prolonged medical benefit compared to pomalidomide+dexamethasone for a substantial fraction of the patients.

### 4.2 Safety Data in Study in Patients with RRMM at time point (bii)

By the data cut-off point for safety data (time point (bii)) 40 patients has been dosed with 183 doses of melflufen hydrochloride 40 mg over 30 minutes. A total of 11 patients had dose reductions from 40 mg to 25 mg melflufen during the study. All dose reductions were in connection with AEs of thrombocytopenia/neutropenia. Seven (7) patients (64%) had dose reductions in connection to thrombocytopenia, 3 patients to neutropenia (27%) and 1 patient (9%) to both thrombocytopenia and neutropenia.

At time point (bii), thirty-six (36) of the 40 patients treated had discontinued from treatment for reasons described in Table 11, while 4 patients are still ongoing in the trial. Eighteen (18) patients had discontinued trial treatment due to AEs. 4 patients were still in treatment, 3 had completed treatment and 33 patients discontinued from treatment (18 due to AEs, 12 due to PD, 2 deaths and 1 for other reasons). 29 patients were still in the study (4 patients in treatment and 25 in follow-up), while 11 patients were off study (8 patients due to death, 1 due to PD, 1 withdrew consent and 1 lost in follow-up).

**Table 11: Disposition among Patients Dosed with 40 mg Melflufen (N=40), Treatment Duration and Relation to Response in Example 2b**

| Disposition | Number of patients | Reason for discontinuation | n |
|---|---|---|---|
| Ongoing on treatment | 4 | | |
| Discontinued treatment | 36 | Completed study (≥ 8 cycles of therapy) | 3 |
| | | Adverse Events^{a} | 18 |
| | | Death | 2 |
| | | Progressive disease | 12 |
| | | Cachexia in progressive disease | 1 |
| Discontinued study in follow-up | 11 | Lost to follow-up | 1 |
| | | Progressive disease | 1 |
| | | Withdrew consent | 1 |
| | | Death | 8 |
| Remain alive and in follow-up | 25 | | |
| ^{a} Some patients discontinued due to more than one adverse event and are therefore included in more than one subcategory: thrombocytopenia 12, neutropenia/febrile neutropenia 3, fever 2, anemia 2, diarrhoea 1, hypercalcemia 1, unrelated infection 1. | | | |

The median number of cycles initiated was 4 (1-14) and the median duration of treatment was 16.1 weeks (3-61). The 11 patients with dose reductions received a total of 37 cycles of therapies after dose reduction to 25 mg of melflufen [median 3 cycles [range 1 to 8 cycles]).

The mean dose intensity in patients without dose reduction (N=29) was 3.58 mg/day; the mean dose intensity with patients with dose reductions to 25 mg (N=11) was 1.31 mg/day while on 40 mg and 0.72 mg/day while on 25 mg. The duration of melflufen treatment and mean dose intensity is presented in Table 12.

**Table 12: Summary of Exposure to Melflufen in in Example 2b (N=40)**

| | |
|---|---|
| Total number of doses given | 183 |
| Median number of cycles per patient (range) | 4 (1-14) |
| Median duration of treatment (range) | 16.1 weeks (3 to 61) |
| Median cumulative dose per patient (range) | 120 mg (40 to 440 mg) |
| Mean dose intensity in patients without dose | 1.58 mg/day |
| Mean dose intensity in patients with dose reductions (N=11)^{a} | 1.31 mg/day |
| ^{a} Mean dose intensity was calculated as total given dose divided by the number of days of completed cycles. | |

The most frequent treatment emerging adverse events (TEAEs) occurring at least once in a specific patient in the group (including all grades and regardless of relationship to study drug) included thrombocytopenia (73%), anemia (65%), neutropenia (65%), pyrexia (43%), asthenia (35%), nausea (28%) and diarrhoea (25%).

34 (85%) patients experienced treatment-related grade 3 or 4 TEAEs. The most common treatment related Grade 3 and 4 TEAEs were bone marrow related, such as reversible thrombocytopenia and neutropenia, which occurred at least once in 63% and 58% of the 40 mg treated patients, respectively. Other common events included anemia (43%). Hyperglycemia has been reported as treatment-related to dexamethasone where Grade 3/4 hyperglycemia has occurred in 4 patients (10%) of the 40 mg treated patients with 1 patient experiencing Grade 4 hyperglycemia. The incidence of Grade 3-4 pneumonia was 14%. For comparison, the corresponding incidence for pomalidomide + dexamethasone is 16% according to the Pomalyst label (FDA Pomalyst label (2015) http://wwwaccessdatafdagov/drugsatfda_docs/label/2015/204026s005s006s008lbI.pdf).

The incidences of treatment related ≥ Grade 3 and Grade 4 TEAEs reported in >5% of patients (n=40) receiving melflufen hydrochloride 40 mg throughout all treatment cycles are shown in Table 13.

Also provided in Table 13 are a summary of the Grade ≥3 and Grade 4 TEAEs in 6 patients dosed with a higher melflufen hydrochloride dose (55 mg) in Example 1. The TEAEs are assessed as related to the study treatment. In the 55 mg melflufen hydrochloride dose group, all patients developed Grade 3 or Grade 4 neutropenia and 5 patients out of 6 patients developed Grade 3 or Grade 4 thrombocytopenia. As shown in Table 13, the incidences of thrombocytopenia and neutropenia were much lower for the 40 mg melflufen hydrochloride dose.

The occurrences of other ≥Grade 3 and Grade 4 TEAEs (i.e. excluding thrombocytopenia and neutropenia) were low for the 40 mg dosage of melflufen hydrochloride. Hematologic toxicity was common, but non-hematologic TEAEs were infrequent.

It was found that the safety profile for melflufen is similar to that for other alkylators, with neutropenia and thrombocytopenia as the most common AEs.

**Table 13: Treatment Related ≥ Grade 3 TEAEs Reported in >5% of Patients (N=40) in Example 2b**

| System Organ Class *(Preferred Term)* | | 40 mg melflufen hydrochloride | | 55 mg melflufen hydrochloride |
|---|---|---|---|---|
| | | Treatment related ≥grade 3 n (%) | Treatment related grade 4 n (%) | Treatment related ≥grade 3 n (%) |
| Any treatment-related grade 3 and/or grade 4 | | 34 (85) | 20 (50) | 6 (100) |
| Blood and lymphatic system disorders | | 33 (82.5) | 20 (50) | 6 (100) |
| | *Thrombocytopenia* | 25 (62.5) | 16 (40) | 5 (83) |
| | *Neutropenia* | 23 (57.5) | 12 (30) | 6 (100) |
| | *Anemia* | 17 (43) | 0 | 2 (33) |
| | *Febrile neutropenia* | 2 (5) | 0 | 0 |
| | *Pancytopenia* | 0 | 0 | 1 (17) |
| General disorders and administration site conditions | | 7 (17.5) | 0 | 1 (17) |
| | *Asthenia* | 2 (5) | 0 | 0 |
| | *Fatigue* | 2 (5) | 0 | 1 (17) |
| | *Pyrexia* | 2 (5) | 0 | 0 |
| Infections and infestations | | 2 (5) | 0 | 3 (50) |
| | *Pneumonia* | 2 (5) | 0 | 2 (33) |
| | *Sepsis* | | | 1 (17) |
| | *Urinary tract infection* | | | 1 (17) |
| Investigations | | 5 (12.5) | 0 | 0 |
| | *Neutrophil count decreased* | 4 (10) | 0 | 0 |
| *White blood cell count decreased* | | 2 (5) | 0 | 0 |
| Metabolism and nutrition disorders | | 3 (8) | 1 (3) | 0 |
| | *Hyperglycemia* | 3 (8) | 1 (3) | 0 |
| Skin and subcutaneous disorders | | 0 | 0 | 1 (17) |
| *Cutaneous rash* | | 0 | 0 | 1 (17) |

23 patients of the 40 mg melflufen (+dex) treated patients had reported Grade 3 and 4 neutropenia, 10% pneumonia, 5% febrile neutropenia and 2% (1 patient) each reported lower respiratory tract infection and parainfluenza virus infection regardless of relationship to study treatment. Comparative data from the pomalidomide + dexamethasone arm in the pomalidomide phase 3 study (FDA Pomalyst Prescribing Information (2015): (FDA Pomalyst label (2015) http://www.accessdata.fda.gov/drugsatfda_docs/label/2015/204026s005s006s008lbI.pdf) showed 48% neutropenia, 16% pneumonia, 3% upper respiratory infections and 1% neutropenic sepsis as Grade 3 and 4 events. The Grade 3 and 4 AE rate with respect to neutropenia and infections was similar between the two studies. A total of 6 of the 57 patients (11%) in the ongoing melflufen study experienced fatal events, while on treatment or within 30 days of last dose, compared with 13% in the pomalidomide phase 3 study (EPAR data).

16 patients (40%) experienced Serious TEAEs and 12 patients (30%) experienced treatment-related Serious TEAEs (Table 14). 11 patients (32.5%) had TEAEs leading to dose reduction of melflufen from 40 mg to 25 mg. An overview of dose modifications due to AEs (interruptions and reductions) is provided in Table 15.

3 patients (8%) had infectious adverse effects with fatal outcomes that were possibly related to the study treatments.

**Table 14 Serious TEAEs Related to Melflufen 40 mg in Example 2b**

| Adverse Event Term | Number of patients (%) |
|---|---|
| Pneumonia | 4 (10) |
| Febrile Neutropenia | 2 (5) |
| Pyrexia | 2 (5) |
| Diarrhoea | 2 (5) |
| Escherichia coli sepsis | 1 (2.5) |
| Neutropenia | 2 (5) |

**Table 15: Dose Interruptions and Reductions in Patients Receiving the Dose 40 mg (N = 40) in Example 2b**

| Cycle length (days) | Total number of patients | Total number of cycles given | Patients with a dose reduction^{a} n (% of total) | Patients with a dose interruption^{a} n (% of total) | Cycles with a dose interruption^{a} n (% of total) |
|---|---|---|---|---|---|
| 21 | 30 | 87 | 11 (37) | 17 (57) | 32 (37) |
| 28 | 10 | 24 | 2 (20) | 3 (30) | 3 (13) |
| ^{a} 10 patients only received one dose due to early progressive disease (PD) and have by definition, no dose interruption or reduction. Dose interruption is defined as a delay ≥ 1 week. | | | | | |

Prolongation of the cycle length from 21 days to 28 days lead to a decrease in the proportion of patients with a dose reduction or a dose interruption, as shown in Table 14. Prolongation of the cycle length from 21 days to 28 days also substantially decreased the incidence of dose interruptions.

Finally, treatment discontinuations due to treatment-related bone marrow suppression occurred in 14 patients of the 40 safety evaluable patients (35%) after a median of 3.5 cycles with thrombocytopenia as the most common event. Ten (10) of these 14 patients received the dose 40 mg throughout the study until treatment discontinuation.

### 5 Discussion of safety data

The clinical trials indicate that the safety profile for melflufen administered in accordance with the invention is similar to that for other alkylators, where neutropenia and thrombocytopenia are the most common AEs, followed by anemia and leukopenia. The incidences of Grade 3 and 4 neutropenia and thrombocytopenia after 40 mg doses of melflufen administered over 30 minutes are comparable to the incidences observed in studies with low-dose melphalan regimens in combination with high dose steroids (Richardson, P., et al. British Journal of Haematology (2011) Vol 153, pages 212 - 221). There have been no reports of syncope, seizures, ventricular arrhythmias, ventricular tachycardia, ventricular fibrillation, flutter, torsade de pointes, or sudden deaths in the clinical trials. The combined data indicate that favourable efficacy results for melflufen administered in accordance with the invention, as described in Sections 3.2 and 4.2, above, have been observed with no increase in toxicity when compared to other alkylating agents.

## Claims

1. Melflufen, or a salt thereof, for use in the treatment of multiple myeloma,
wherein a 37.5 to 42.5 mg dosage of melflufen, excluding the mass of any salt, is administered as a parenteral dosage at an infusion rate of 1.2 to 1.4 mg/min;
and wherein the dosage of melflufen, or a salt thereof, is taken on day 1 of a cycle of 28 days.

2. The compound for use as claimed in claim 1, wherein the infusion rate is 1.3 mg/min.

3. The compound for use as claimed in claim 1 or 2, wherein the dosage is administered over 25 to 35 minutes.

4. The compound for use as claimed in any one of claims 1 to 3, wherein the dosage of melflufen, excluding the mass of any salt, is 40 mg.

5. The compound for use as claimed in any one of claims 1 to 4, wherein the dosage of melflufen is administered over around 30 minutes.

6. The compound for use as claimed in any one of claims 1 to 5, wherein the dosage of melflufen is administered as an intravenous infusion.

7. The compound for use as claimed in any one of claims 1 to 6, in which the multiple myeloma is relapsed, refractory and/or relapsed refractory multiple myeloma, for example refractory to at least one drug from a class of drugs selected from protease inhibitors, immunomodulatory drugs or alkylators; for example at refractory to least one alkylator; and/or, for example, refractory to at least pomalidomide and/or daratumumab.

8. The compound for use as claimed in in any one of claims 1 to 7, in which the multiple myeloma is relapsed and/or relapsed refractory to at least lenalidomide; and more especially to at least lenalidomide and 2, 3 or 4 other drugs including at least one protease inhibitors and immunomodulatory drugs.

9. The compound for use as claimed in any one of claims 1 to 8, in which said melflufen is administered simultaneously, sequentially or separately with one or more further therapeutic agent(s), for example wherein said further therapeutic agent is dexamethasone.

10. The compound for use as claimed in any one of claims 1 to 9, wherein the dosage of melflufen is administered as a pharmaceutical solution having a volume of 200 to 500 ml, preferably 350 ml.

11. The compound for use as claimed in any one of claims 1 to 10, wherein the dosage of melflufen is administered as a pharmaceutical solution comprising a physiologically acceptable solution, for example a glucose solution.

12. The compound for use as claimed in any one of claims 1 to 11, wherein the dosage of melflufen is administered as a pharmaceutical solution and wherein the concentration of melflufen, or a salt thereof, in the pharmaceutical solution is 1.2 mg/mL or less, for example 0.2 to 1.2 mg/mL.

13. The compound for use as claimed in any one of claims 1 to 12, wherein the dosage of melflufen is prepared from a lyophilized pharmaceutical preparation comprising melflufen, or a salt thereof, and optionally sucrose.

14. The compound for use as claimed in any one of claims 1 to 13, wherein the dosage of melflufen, or a salt thereof, is taken on day 1 of a cycle of 28 days, and wherein the cycle is repeated from 1 to 9 times, preferably from 2 to 7 times, for example 4 times.

15. The compound for use as claimed in any one of claims 1 to 14, wherein dexamethasone is administered on day 1 in a cycle; and optionally also administered on days 8, 15 and 22 of a 28 day cycle.

## Patentansprüche

1. Melflufen oder ein Salz davon, zur Verwendung bei der Behandlung von multiplem Myelom, wobei eine 37,5 bis 42,5 mg Dosis von Melflufen, ohne die Masse von jeglichem Salz, als parenterale Dosis mit einer Infusionsrate von 1,2 bis 1,4 mg / min verabreicht wird, und wobei die Dosis von Melflufen oder einem Salz davon am Tag 1 von einem Zyklus von 28 Tagen eingenommen wird.

2. Verbindung zur Verwendung nach Anspruch 1, wobei die Infusionsrate 1,3 mg / min beträgt.

3. Verbindung zur Verwendung nach Anspruch 1 oder 2, wobei die Dosis über 25 bis 35 min verabreicht wird.

4. Verbindung zur Verwendung nach einem der Ansprüche 1 bis 3, wobei die Dosis von Melflufen, ohne die Masse von jeglichem Salz, 40 mg beträgt.

5. Verbindung zur Verwendung nach einem der Ansprüche 1 bis 4, wobei die Dosis von Melflufen über ca. 30 Minuten verabreicht wird.

6. Verbindung zur Verwendung nach einem der Ansprüche 1 bis 5, wobei die Dosis von Melflufen als intravenöse Infusion verabreicht wird.

7. Verbindung zur Verwendung nach einem der Ansprüche 1 bis 6, in der das multiple Myelom ein rezidiviertes, refraktäres und / oder rezidiviertes refraktäres multiples Myelom, beispielsweise refraktär gegen mindestens ein Arzneimittel aus einer Klasse von Arzneimitteln ist, ausgewählt aus Proteaseinhibitoren, immunmodulatorischen Arzneimitteln oder Alkylantien; zum Beispiel refraktär gegen mindestens ein Alkylans; und / oder zum Beispiel refraktär gegen mindestens Pomalidomid und / oder Daratumumab.

8. Verbindung zur Verwendung nach einem der Ansprüche 1 bis 7, bei der das multiple Myelom rezidiviert und / oder refraktär gegen mindestens Lenalidomid ist; und insbesondere gegen mindestens Lenalidomid und 2, 3 oder 4 andere Arzneimittel, einschließlich mindestens einen Proteaseinhibitor und immunmodulatorische Arzneimittel.

9. Verbindung zur Verwendung nach einem der Ansprüche 1 bis 8, bei der das Melflufen gleichzeitig, nacheinander oder getrennt mit einem oder mehreren weiteren therapeutischen Mitteln verabreicht wird, beispielsweise wobei das weitere therapeutische Mittel Dexamethason ist.

10. Verbindung zur Verwendung nach einem der Ansprüche 1 bis 9, wobei die Dosis von Melflufen als pharmazeutische Lösung mit einem Volumen von 200 bis 500 ml, vorzugsweise 350 ml, verabreicht wird.

11. Verbindung zur Verwendung nach einem der Ansprüche 1 bis 10, wobei dieDosis von Melflufen als pharmazeutische Lösung verabreicht wird, die eine physiologisch verträgliche Lösung, beispielsweise eine Glucoselösung, umfasst.

12. Verbindung zur Verwendung nach einem der Ansprüche 1 bis 11, worin dieDosis von Melflufen als pharmazeutische Lösung verabreicht wird und wobei die Konzentration von Melflufen oder eines Salzes davon in der pharmazeutischen Lösung 1,2 mg / ml oder weniger, beispielsweise 0,2 bis 1,2 mg / ml beträgt.

13. Verbindung zur Verwendung nach einem der Ansprüche 1 bis 12, worin die Dosis von Melflufen aus einem lyophilisierten pharmazeutischen Präparat hergestellt wird, das Melflufen oder ein Salz davon und gegebenenfalls Saccharose umfasst.

14. Verbindung zur Verwendung nach einem der Ansprüche 1 bis 13, wobei die Dosis von Melflufen oder eines Salzes davon am Tag 1 von einem Zyklus von 28 Tagen eingenommen wird und wobei der Zyklus 1 bis 9 mal, vorzugsweise 2 bis 7 mal, beispielsweise 4 mal wiederholt wird.

15. Verbindung zur Verwendung nach einem der Ansprüch 1 bis 14, wobei Dexamethason am Tag 1 in einem Zyklus verabreicht wird; und optional auch an den Tagen 8, 15 und 22 eines 28-Tages-Zyklus verabreicht wird.

## Revendications

1. Melflufen, ou sel de celui-ci, destiné à être utilisé dans le traitement du myélome multiple, où une dose de melflufen de 37,5 à 42,5 mg, excluant la masse d'un quelconque sel, est administrée sous forme d'une dose parentérale à un débit de perfusion de 1,2 à 1,4 mg/min,
et dans lequel la dose de melflufen, ou d'un de ses sels, est prise au jour 1 d'un cycle de 28 jours.

2. Composé destiné à être utilisé selon la revendication 1, dans lequel le débit de perfusion est de 1,3 mg/min.

3. Composé destiné à être utilisé selon la revendication 1 ou 2, dans lequel la dose est administrée pendant 25 à 35 minutes.

4. Composé destiné à être utilisé selon l'une quelconque des revendications 1 à 3, dans lequel la dose de melflufen, excluant la masse d'un quelconque sel, est de 40 mg.

5. Composé destiné à être utilisé selon l'une quelconque des revendications 1 à 4, dans lequel la dose de melflufen est administrée pendant environ 30 minutes.

6. Composé destiné à être utilisé selon l'une quelconque des revendications 1 à 5, dans lequel la dose de melflufen est administrée sous forme de perfusion intraveineuse.

7. Composé destiné à être utilisé selon l'une quelconque des revendications 1 à 6, dans lequel le myélome multiple est un myélome multiple en rechute, réfractaire et/ou en rechute et réfractaire, par exemple réfractaire à au moins un médicament d'une classe de médicaments sélectionnée parmi des inhibiteurs de protéase, des médicaments immunomodulateurs ou des agents alkylants ; par exemple réfractaire à au moins un agent alkylant ; et/ou, par exemple, réfractaire à au moins le pomalidomide et/ou le daratumumab.

8. Composé destiné à être utilisé selon l'une quelconque des revendications 1 à 7, dans lequel le myélome multiple est en rechute et/ou en rechute et réfractaire à au moins le lénalidomide ; et plus particulièrement à au moins le lénalidomide et 2, 3 ou 4 autres médicaments comprenant au moins l'un d'inhibiteurs de protéase et de médicaments immunomodulateurs.

9. Composé destiné à être utilisé selon l'une quelconque des revendications 1 à 8, dans lequel ledit melflufen est administré simultanément, séquentiellement ou séparément avec un ou plusieurs agent(s) thérapeutique(s) supplémentaire(s), par exemple dans lequel ledit agent thérapeutique supplémentaire est la déxaméthasone.

10. Composé destiné à être utilisé selon l'une quelconque des revendications 1 à 9, dans lequel la dose de melflufen est administrée sous forme de solution pharmaceutique possédant un volume de 200 à 500 ml, de préférence 350 ml.

11. Composé destiné à être utilisé selon l'une quelconque des revendications 1 à 10, dans lequel la dose de melflufen est administrée sous forme de solution pharmaceutique comprenant une solution physiologiquement acceptable, par exemple une solution de glucose.

12. Composé destiné à être utilisé selon l'une quelconque des revendications 1 à 11, dans lequel la dose de melflufen est administrée sous forme de solution pharmaceutique et dans lequel la concentration de melflufen, ou d'un sel de celui-ci, dans la solution pharmaceutique est de 1,2 mg/ml ou moins, par exemple 0,2 à 1,2 mg/ml.

13. Composé destiné à être utilisé selon l'une quelconque des revendications 1 à 12, dans lequel la dose de melflufen est préparée à partir d'une préparation pharmaceutique lyophilisée comprenant du melflufen, ou un sel de celui-ci, et facultativement du saccharose.

14. Composé destiné à être utilisé selon l'une quelconque des revendications 1 à 13, dans lequel la dose de melflufen, ou d'un sel de celui-ci, est prise au jour 1 d'un cycle de 28 jours, et dans lequel le cycle est répété de 1 à 9 fois, de préférence de 2 à 7 fois, par exemple 4 fois.

15. Composé destiné à être utilisé selon l'une quelconque des revendications 1 à 14, dans lequel la déxaméthasone est administrée au jour 1 dans un cycle ; et facultativement est également administrée aux jours 8, 15 et 22 d'un cycle de 28 jours.
